# EUROPEAN PATENT APPLICATION

(11) **EP 3 760 718 A1**
(43) Date of publication of application: **06.01.2021**
(21) Application number: 19184380.4
(22) Date of filing: 04.07.2019
(51) Int. Cl.: C12N 15/113

(54) **TISSUE FOR USE AS ALLOGENEIC OR XENOGENEIC TRANSPLANT AND METHOD FOR ITS PRODUCTION**

(71) Applicant: Medizinische Hochschule Hannover, 30625 Hannover (DE)
(72) Inventor: Blasczyk, Rainer, 30916 Isernhagen (DE); Ferreira de Figueiredo, Constanca, 31535 Neustadt am Rübenberge (DE)
(74) Representative: Taruttis, Stefan Georg

(57) **Abstract**

The invention relates to a tissue for use as a transplant, which tissue is allogeneic or xenogeneic and respectively the tissue may express an MHC I molecule that is immunologically incompatible to the transplant recipient and/or may express an MHC II molecule immunologically incompatible to the transplant recipient. The tissue suitable for use as a transplant and the method for its production comprise a genetic alteration of the tissue that provides for immunologic compatibility of the tissue with a transplant recipient. In the tissue for use as a transplant, which tissue expresses allogeneic or xenogeneic MHC I and/or allogeneic or xenogeneic MHC II molecules, the expression of the allogeneic or xenogeneic MHC I is downregulated by at least 50% to up to 90%, wherein preferably the expression of the allogeneic or xenogeneic MHC I is downregulated by at least 60%.

SEQUENCE LISTING

<110> Medizinische Hochschule Hannover

<120> Tissue for use as allogeneic or xenogeneic transplant and method for
its production

<130> M1087EP

<160> 38

<170> BiSSAP 1.3.6

<210> 1
<211> 47
<212> DNA
<213> Artificial Sequence

<220>
<223> shRNA for human beta2m transcript (NM_004048.2, start nt 244)

<400> 1
gaatggagag agaattgaat tcaagagatt caattctctc tccattc                47

<210> 2
<211> 47
<212> DNA
<213> Artificial Sequence

<220>
<223> Less efficient shRNA sequence for human beta2m transcript:
      HS_sh2m-2 (NM_004048.2, start nt 118)

<400> 2
gctatccagc gtactccaat tcaagagatt ggagtacgct ggatagc                47

<210> 3
<211> 51
<212> DNA
<213> Artificial Sequence

<220>
<223> shRNA for human CIITA: HS_shCIITA (NM_000246, start nt 400)

<400> 3
aagagaccag ggaggcttat gttcaagaga cataagcctc cctggtctct t           51

<210> 4
<211> 49
<212> DNA
<213> Artificial Sequence

<220>
<223> Less efficient shRNA for human CIITA: HS_shCIITA-2 (NM_000246,
      start nt 3469)

<400> 4
gcccattcag tgtccaggaa ttcaagagat tcctggacac tgaatgggc          49

<210> 5
<211> 49
<212> DNA
<213> Artificial Sequence

<220>
<223> Shorter less efficient shRNA for human CIITA: HS_shCIITA-3
      (NM_000246, start nt 400)

<400> 5
aagagaccag ggaggcttat ttcaagagaa taagcctccc tggtctctt          49

<210> 6
<211> 49
<212> DNA
<213> Artificial Sequence

<220>
<223> shRNA sequence for swine (Sus Scrofa) beta2m: SS_sh2m (L13854.1,
      start nt 3471)

<400> 6
cacgtgactc tcgataagcc ttcaagagag gcttatcgag agtcacgtg          49

<210> 7
<211> 49
<212> DNA
<213> Artificial Sequence

<220>
<223> Mismatched and less efficient shRNA sequence for swine (Sus
      Scrofa) 2m: SS_sh2m-2 (L13854.1, start nt 3471)

<400> 7
cacgtgacac tcgataagcc ttcaagagag gcttatcgag tgtcacgtg          49

<210> 8
<211> 49
<212> DNA
<213> Artificial Sequence

<220>
<223> shRNA sequence for swine (Sus Scrofa) CIITA: SS_shCIITA
      (XM_005662139.1, start nt1173)

<400> 8
ctgccacagt acgactttgt ttcaagagaa caaagacgta ctgtggcag          49

<210> 9
<211> 47
<212> DNA

<213> Artificial Sequence

<220>
<223> shRNA sequence for rat (Rattus norvegicus) beta2m: RN_2m
      (NM_012512.2, start nt188 )

<400> 9
ggaaagaaga taccaaatat tcaagagata tttggtatct tctttcc                47

<210> 10
<211> 51
<212> DNA
<213> Artificial Sequence

<220>
<223> shRNA sequence for rat (Rattus norvegicus) CIITA: RN_shCIITA
      (AF251307.1, start nt 176)

<400> 10
ggatatggaa atggatgaag attcaagaga tcttcatcca tttccatatc c           51

<210> 11
<211> 245
<212> DNA
<213> Artificial Sequence

<220>
<223> Elongation Factor short (EFS) promoter (driving expression of
      reporter gene)

<400> 11
tggctccggt gcccgtcagt gggcagagcg cacatcgccc acagtccccg agaagttggg    60

gggaggggtc ggcaattgaa ccggtgccta gagaaggtgg cgcggggtaa actgggaaag   120

tgatgtcgtg tactggctcc gcctttttcc cgagggtggg ggagaaccgt atataagtgc   180

agtagtcgcc gtgaacgttc tttttcgcaa cgggtttgcc gccagaacac aggtgtcgtg   240

acgcg                                                               245

<210> 12
<211> 1080
<212> DNA
<213> Artificial Sequence

<220>
<223> EF1alpha promoter (driving expression of reporter gene)

<400> 12
catttcaggt gtcgtgagga atttcgacat ttaaatttaa ttaatctcga cggtatcggt    60

taacttttaa aagaaaaggg gggattgggg ggtacagtgc aggggaaaga atagtagaca   120

taatagcaac agacatacaa actaaagaat tacaaaaaca aattacaaaa attcaaaatt   180

ttatcgatca cgagactagc ctcgaggttt aaactacggg atcttcgaag gcctaagctt 240

acgcgcgcgt cctagcgcta ccggtcgcca ccatggtgag caagggcgag gagctgttca 300

ccggggtggt gcccatcctg gtcgagctgg acggcgacgt aaacggccac aagttcagcg 360

tgtccggcga gggcgagggc gatgccacct acggcaagct gaccctgaag ttcatctgca 420

ccaccggcaa gctgcccgtg ccctggccca ccctcgtgac caccctgacc tacggcgtgc 480

agtgcttcag ccgctacccc gaccacatga agcagcacga cttcttcaag tccgccatgc 540

ccgaaggcta cgtccaggag cgcaccatct tcttcaagga cgacggcaac tacaagaccc 600

gcgccgaggt gaagttcgag ggcgacaccc tggtgaaccg catcgagctg aagggcatcg 660

acttcaagga ggacggcaac atcctggggc acaagctgga gtacaactac aacagccaca 720

acgtctatat catggccgac aagcagaaga acggcatcaa ggtgaacttc aagatccgcc 780

acaacatcga ggacggcagc gtgcagctcg ccgaccacta ccagcagaac acccccatcg 840

gcgacggccc cgtgctgctg cccgacaacc actacctgag cacccagtcc gccctgagca 900

aagaccccaa cgagaagcgc gatcacatgg tcctgctgga gttcgtgacc gccgccggga 960

tcactctcgg catggacgag ctgtacaagt ccggactcag atctcgacta gctagtagct 1020

agctagctag tcgagctcaa gcttcgggga ctagtcatat gataatcaac ctctggatta 1080

<210> 13
<211> 19
<212> DNA
<213> Artificial Sequence

<220>
<223> Tet-operator element

<400> 13
tccctatcag tgatagaga 19

<210> 14
<211> 220
<212> DNA
<213> Artificial Sequence

<220>
<223> H1 promoter to drive the expression of the shRNAs

<400> 14
aattcgaacg ctgacgtcat caacccgctc caaggaatcg cgggcccagt gtcactaggc 60

gggaacaccc agcgcgcgtg cgccctggca ggaagatggc tgtgagggac aggggagtgg 120

cgccctgcaa tatttgcatg tcgctatgtg ttctgggaaa tcaccataaa cgtgaaatgt 180

ctttggattt gggaatctta taagttctgt atgagaccac 220

<210> 15
<211> 333
<212> DNA
<213> Artificial Sequence

<220>
<223> U6 Sequence to drive the expression of the shRNAs (weaker)

<400> 15
gatcccgcgt cctttccaca agatatataa acccaagaaa tcgaaatact ttcaagttac      60

ggtaagcata tgatagtcca ttttaaaaca taattttaaa actgcaaact acccaagaaa     120

ttattacttt ctacgtcacg tattttgtac taatatcttt gtgtttacag tcaaattaat     180

tctaattatc tctctaacag ccttgtatcg tatatgcaaa tatgaaggaa tcatgggaaa     240

taggccctct tcctgcccga ccttggcgcg cgctcggcgc gcggtcacgc tccgtcacgt     300

ggtgcgtttt gcctgcgcgt ctttccactg ggg                                  333

<210> 16
<211> 63
<212> DNA
<213> Artificial Sequence

<220>
<223> T2A peptide encoding nucleic acid sequence

<220>
<221> CDS
<222> 1..63
<223> /note="T2A peptide"
      /transl_table=1

<400> 16
ggc tcc ggc gag ggc agg gga agt ctt cta aca tgc ggg gac gtg gag    48
Gly Ser Gly Glu Gly Arg Gly Ser Leu Leu Thr Cys Gly Asp Val Glu
1               5                   10                  15

gaa aat ccc ggc cca                                                63
Glu Asn Pro Gly Pro
            20

<210> 17
<211> 1497
<212> DNA
<213> Artificial Sequence

<220>
<223> SS_CD55 (SS = Sus Scrofa = pig)

<400> 17
atgagtcccc tgccgcggag cgcccccgcg gtgaggcgcc taatgggcgg acagacgccg      60

ccgccgctgc tgctgctgct gctgctgctg tgtatcccgg ctgcgcaggg tgactgcagc     120

cttccacccg atgtacctaa tgcccaacca gatttgcgag gtcttgcaag ttttcctgaa     180

caaaccacaa taacatacaa atgtaacaaa ggctttgtca aagttcctgg catggcagac 240

tcagtgctct gtcttaatga taaatggtca gaagttgcag aattttgtaa tcgtagctgt 300

gatgttccaa ccaggctaca ttttgcatct cttaaaaagt cttacagcaa acagaattat 360

ttcccagagg gtttcaccgt ggaatatgag tgccgtaagg gctataaaag ggatcttact 420

ctatcagaaa aactaacttg ccttcagaat tttacgtggt ccaaacctga tgaattttgc 480

aaaaaaaaac aatgtccgac tcctggagaa ctaaaaaatg gtcatgtcaa tataacaact 540

gacttgttat ttggcgcatc catctttttc tcatgtaacg cagggtacag actagttggt 600

gcaacttcta gttactgttt tgccatagca aatgatgttg agtggagtga tccattgcca 660

gattgccaag aaatttctcc aactgtcaaa gccataccag ctgttgagaa acccatcaca 720

gtaaattttc caggtaccaa agccctatca tctcctcaga aaccctccac agcaaatact 780

ctagctacag agttactacc aactcctcag gaacccacca cagtaaatgt tccagatagt 840

aaagccatat catctcctca gaaaccctcc acagtaaata ctccagctac agacttacta 900

ccaactcctc aggaacccac cacagtaaat gttccagata gtaaagccat atcatcttct 960

cagaaaccct ccacagtaaa tactccagct acagacttac taccaactcc tcaggaaccc 1020

accacagtaa atgttccaga tagtaaagcc atatcatctc ctcagaaacc ctccacagta 1080

aatactccag ctacagactt actaccaact cctcaggaac ccaccacagt aaatgttcca 1140

gctccagaag ttccatcaac ttctcagaaa ccttccacag caaatgattc tgcaccacag 1200

gccacatcaa caacccaaag attcaccaca gcaaaagctt cactgacaca gactcttcga 1260

gaaacaggaa agtccactcc tgtacatacc tcaaaaacta aaggtctaca gcaaagattg 1320

acttctgctc atgttacagc aacaaagtat ccagctattc ccagggccac aacgagtttt 1380

cattcaagta catctaaaaa tcgaggaaac ccttcttcag gcatgagaat catgtcgtct 1440

ggacgtgcat gtgtaacact gacagttttg cttatggtgc tagtaaccat tggctag 1497

<210> 18
<211> 1401
<212> DNA
<213> Artificial Sequence

<220>
<223> RN_CD55 (RN = Ratus Norvegicus = Rat)

<220>
<221> CDS
<222> 1..1401
<223> /note="rat CD55"
/transl_table=1

<400> 18
atg atc cgc acg cgg gcg ccg ggg act cgg gct ccg ccg ccg ccg ccg 48
Met Ile Arg Thr Arg Ala Pro Gly Thr Arg Ala Pro Pro Pro Pro Pro

1                 5                   10                  15

ctg ctg ctg tcg ctg tcg ctg ctg ctg ctg ctg ctg tcc ccg act gta        96
Leu Leu Leu Ser Leu Ser Leu Leu Leu Leu Leu Leu Ser Pro Thr Val
            20                  25                  30

agc gga gac tgc ggc cca cct cca gac att cct aat gcc acg cca aac       144
Ser Gly Asp Cys Gly Pro Pro Pro Asp Ile Pro Asn Ala Thr Pro Asn
        35                  40                  45

tcg ggt aaa cac acc aag ttt gcc cac caa agc caa gtg aca tat tcg       192
Ser Gly Lys His Thr Lys Phe Ala His Gln Ser Gln Val Thr Tyr Ser
    50                  55                  60

tgt aat aag ggc ttc aaa caa att cca gac aag cca aac aca gtg gtc       240
Cys Asn Lys Gly Phe Lys Gln Ile Pro Asp Lys Pro Asn Thr Val Val
65                  70                  75                  80

tgc ctt gaa aat gac caa tgg tct aac tac gaa act ttc tgt aat aaa       288
Cys Leu Glu Asn Asp Gln Trp Ser Asn Tyr Glu Thr Phe Cys Asn Lys
                85                  90                  95

acc tgt agt gct cca ctg aga ctg aat tat gca tcc ctc aaa aag gag       336
Thr Cys Ser Ala Pro Leu Arg Leu Asn Tyr Ala Ser Leu Lys Lys Glu
            100                 105                 110

tat cgt aac atg aat ttt ttc cca att ggc acc act gtg gaa ttt gag       384
Tyr Arg Asn Met Asn Phe Phe Pro Ile Gly Thr Thr Val Glu Phe Glu
        115                 120                 125

tgt cgg cca gga ttt caa aaa gta act tca cgc aca gga aaa tca act       432
Cys Arg Pro Gly Phe Gln Lys Val Thr Ser Arg Thr Gly Lys Ser Thr
    130                 135                 140

tgc ctt gag gaa tta gta tgg tct cca gtt gtt gaa ttt tgt aaa aaa       480
Cys Leu Glu Glu Leu Val Trp Ser Pro Val Val Glu Phe Cys Lys Lys
145                 150                 155                 160

aga gca tgc cct aat cct aga gaa ctg aaa aat ggt cac atc aac ata       528
Arg Ala Cys Pro Asn Pro Arg Glu Leu Lys Asn Gly His Ile Asn Ile
                165                 170                 175

cca act gac ata tta ttc gga tca gaa ata atc ttc tca tgc gac aaa       576
Pro Thr Asp Ile Leu Phe Gly Ser Glu Ile Ile Phe Ser Cys Asp Lys
            180                 185                 190

ggg tac aag ctg gtc ggt gta act tct aca ttc tgt tct att acg ggg       624
Gly Tyr Lys Leu Val Gly Val Thr Ser Thr Phe Cys Ser Ile Thr Gly
        195                 200                 205

aat gct gtg gac tgg gat gac gta ttt cca gtg tgc aca aca att ttt       672
Asn Ala Val Asp Trp Asp Asp Val Phe Pro Val Cys Thr Thr Ile Phe
    210                 215                 220

tgt cca gac cca cca aaa atc aat aat gga aga att cga gag gaa agc       720
Cys Pro Asp Pro Pro Lys Ile Asn Asn Gly Arg Ile Arg Glu Glu Ser
225                 230                 235                 240

gac tcc tac gca tat aga cag tcg gtg acc tat tcc tgt gac caa ggc       768
Asp Ser Tyr Ala Tyr Arg Gln Ser Val Thr Tyr Ser Cys Asp Gln Gly
                245                 250                 255

ttc att ctg gtt gga aat tct tcc gtt cat tgt act ctg aac ggt gaa       816

Phe Ile Leu Val Gly Asn Ser Ser Val His Cys Thr Leu Asn Gly Glu
            260                 265                 270

gta gga gaa tgg agt agt gca cca ccc aag tgc ata gag aga cac aag      864
Val Gly Glu Trp Ser Ser Ala Pro Pro Lys Cys Ile Glu Arg His Lys
        275                 280                 285

gtc cca act aag acc cca cca gaa gtt gat att cca agt aca atg cct      912
Val Pro Thr Lys Thr Pro Pro Glu Val Asp Ile Pro Ser Thr Met Pro
    290                 295                 300

cat gaa tcc acc tta att aat ttt cca agt aca aga gcc cca ctg tct      960
His Glu Ser Thr Leu Ile Asn Phe Pro Ser Thr Arg Ala Pro Leu Ser
305                 310                 315                 320

cat aaa ccc act aca gtt aaa gtt cca gca aca cag cat gta cct gtt     1008
His Lys Pro Thr Thr Val Lys Val Pro Ala Thr Gln His Val Pro Val
                325                 330                 335

tcc aag aca acg gta cgt cat cca acg aga aca tct aaa gac aga gga     1056
Ser Lys Thr Thr Val Arg His Pro Thr Arg Thr Ser Lys Asp Arg Gly
            340                 345                 350

gag tct aat tca ggt ggt gac cat ttt ata tat gga aag ttt gac cca     1104
Glu Ser Asn Ser Gly Gly Asp His Phe Ile Tyr Gly Lys Phe Asp Pro
        355                 360                 365

cag gga gtt aaa aga aat tgc cat cac tgt ggg aga cat agt cca tat     1152
Gln Gly Val Lys Arg Asn Cys His His Cys Gly Arg His Ser Pro Tyr
    370                 375                 380

ttc tgg aag aga atc ttc tct tat aac tgc ctg aga aaa act atc aaa     1200
Phe Trp Lys Arg Ile Phe Ser Tyr Asn Cys Leu Arg Lys Thr Ile Lys
385                 390                 395                 400

atg cag tgc cta tgg gtg cct aag ggc aga tgg gta aag cct ttt aat     1248
Met Gln Cys Leu Trp Val Pro Lys Gly Arg Trp Val Lys Pro Phe Asn
                405                 410                 415

cgg ctg gtt ttt tgg aca ccg tta ggc atc cag cat ggt cct tca tat     1296
Arg Leu Val Phe Trp Thr Pro Leu Gly Ile Gln His Gly Pro Ser Tyr
            420                 425                 430

ccc caa cag tgc tcc tgc tct aag gca gtc tta gga cag ctt cgg gtc     1344
Pro Gln Gln Cys Ser Cys Ser Lys Ala Val Leu Gly Gln Leu Arg Val
        435                 440                 445

atc tgg cca atc agt cag gta gca ctg agt gcc cgc tgt gtc agt gtg     1392
Ile Trp Pro Ile Ser Gln Val Ala Leu Ser Ala Arg Cys Val Ser Val
    450                 455                 460

tca ttt tag                                                         1401
Ser Phe
465

<210> 19
<211> 372
<212> DNA
<213> Artificial Sequence


<220>
<223> SS_CD59 of Sus scrofa coding sequence

<400> 19
atgggaagca aaggagggtt cattttgctc tggctcctgt ccatcctggc tgttctctgc      60

cacttaggtc acagcctgca gtgctataac tgtatcaacc cagctggtag ctgcactacg     120

gccatgaatt gttcacataa tcaggatgcc tgtatcttcg ttgaagccgt gccacccaaa     180

acttactacc agtgttggag gttcgatgaa tgcaatttcg atttcatttc gagaaaccta     240

gcggagaaga agctgaagta caactgctgc cggaaggacc tgtgtaacaa gagtgatgcc     300

acgatttcat cagggaaaac cgctctgctg gtgatcctgc tgctggtagc aacctggcac     360

ttttgtctct aa                                                         372


<210> 20
<211> 381
<212> DNA
<213> Artificial Sequence


<220>
<223> RN_CD59 rattus norvegicus coding sequence

<220>
<221> CDS
<222> 1..381
<223> /note="CD59 of rattus norvegicus"
      /transl_table=1

<400> 20
atg aga gct cgg agg gga ttc atc tta ctc ctg ctt ctg gct gtc ctc      48
Met Arg Ala Arg Arg Gly Phe Ile Leu Leu Leu Leu Leu Ala Val Leu
1               5                   10                  15

tgt tcc aca ggt gtt agc ctc aga tgc tac aac tgt tta gac ccg gtt      96
Cys Ser Thr Gly Val Ser Leu Arg Cys Tyr Asn Cys Leu Asp Pro Val
            20                  25                  30

tct tca tgc aaa aca aac agc act tgc tct cct aac ctg gat gct tgt     144
Ser Ser Cys Lys Thr Asn Ser Thr Cys Ser Pro Asn Leu Asp Ala Cys
        35                  40                  45

ctt gtt gct gta tcc gga aag caa gtc tat caa cag tgt tgg aga ttt     192
Leu Val Ala Val Ser Gly Lys Gln Val Tyr Gln Gln Cys Trp Arg Phe
    50                  55                  60

tcg gat tgt aat gcc aag ttc att ttg agc cga cta gaa atc gca aac     240
Ser Asp Cys Asn Ala Lys Phe Ile Leu Ser Arg Leu Glu Ile Ala Asn
65                  70                  75                  80

gta caa tac aga tgc tgc cag gcg gac ttg tgt aac aaa agc ttc gaa     288
Val Gln Tyr Arg Cys Cys Gln Ala Asp Leu Cys Asn Lys Ser Phe Glu
                85                  90                  95

gac aag cca aac aat ggg gca atc tcc cta ttg ggg aag aca gcg ttg     336
Asp Lys Pro Asn Asn Gly Ala Ile Ser Leu Leu Gly Lys Thr Ala Leu
            100                 105                 110

ctg gtg acc tcg gtt ctg gcg gcc att ttg aag cct tgt ttc taa         381
Leu Val Thr Ser Val Leu Ala Ala Ile Leu Lys Pro Cys Phe

        115                 120                 125

<210> 21
<211> 126
<212> PRT
<213> Artificial Sequence

<220>
<223> [CDS]:1..381 from SEQ ID NO 20

<400> 21
Met Arg Ala Arg Arg Gly Phe Ile Leu Leu Leu Leu Leu Ala Val Leu
1               5                   10                  15
Cys Ser Thr Gly Val Ser Leu Arg Cys Tyr Asn Cys Leu Asp Pro Val
            20                  25                  30
Ser Ser Cys Lys Thr Asn Ser Thr Cys Ser Pro Asn Leu Asp Ala Cys
        35                  40                  45
Leu Val Ala Val Ser Gly Lys Gln Val Tyr Gln Gln Cys Trp Arg Phe
    50                  55                  60
Ser Asp Cys Asn Ala Lys Phe Ile Leu Ser Arg Leu Glu Ile Ala Asn
65                  70                  75                  80
Val Gln Tyr Arg Cys Cys Gln Ala Asp Leu Cys Asn Lys Ser Phe Glu
                85                  90                  95
Asp Lys Pro Asn Asn Gly Ala Ile Ser Leu Leu Gly Lys Thr Ala Leu
            100                 105                 110
Leu Val Thr Ser Val Leu Ala Ala Ile Leu Lys Pro Cys Phe
        115                 120                 125

<210> 22
<211> 363
<212> PRT
<213> Artificial Sequence

<220>
<223> SS_CD46 of sus scrofa

<400> 22
Met Met Ala Phe Cys Ala Leu Arg Lys Ala Leu Pro Cys Arg Pro Glu
1               5                   10                  15
Asn Pro Phe Ser Ser Arg Cys Phe Val Glu Ile Leu Trp Val Ser Leu
            20                  25                  30
Ala Leu Val Phe Leu Leu Pro Met Pro Ser Asp Ala Cys Asp Glu Pro
        35                  40                  45
Pro Lys Phe Glu Ser Met Arg Pro Gln Phe Leu Asn Thr Thr Tyr Arg
    50                  55                  60
Pro Gly Asp Arg Val Glu Tyr Glu Cys Arg Pro Gly Phe Gln Pro Met
65                  70                  75                  80
Val Pro Ala Leu Pro Thr Phe Ser Val Cys Gln Asp Asp Asn Thr Trp
                85                  90                  95
Ser Pro Leu Gln Glu Ala Cys Arg Arg Lys Ala Cys Ser Asn Leu Pro
            100                 105                 110
Asp Pro Leu Asn Gly Gln Val Ser Tyr Pro Asn Gly Asp Met Leu Phe
        115                 120                 125
Gly Ser Lys Ala Gln Phe Thr Cys Asn Thr Gly Phe Tyr Ile Ile Gly
    130                 135                 140
Ala Glu Thr Val Tyr Cys Gln Val Ser Gly Asn Val Met Ala Trp Ser
145                 150                 155                 160
Glu Pro Ser Pro Leu Cys Glu Lys Ile Leu Cys Lys Pro Pro Gly Glu
                165                 170                 175
Ile Pro Asn Gly Lys Tyr Thr Asn Ser His Lys Asp Val Phe Glu Tyr
            180                 185                 190

Asn Glu Val Val Thr Tyr Ser Cys Leu Ser Ser Thr Gly Pro Asp Glu
        195                 200                 205
Phe Ser Leu Val Gly Glu Ser Ser Leu Phe Cys Ile Gly Lys Asp Glu
    210                 215                 220
Trp Ser Ser Asp Pro Pro Glu Cys Lys Val Val Lys Cys Pro Tyr Pro
225                 230                 235                 240
Val Val Pro Asn Gly Glu Ile Val Ser Gly Phe Gly Ser Lys Phe Tyr
                245                 250                 255
Tyr Lys Ala Glu Val Val Phe Lys Cys Asn Ala Gly Phe Thr Leu His
            260                 265                 270
Gly Arg Asp Thr Ile Val Cys Gly Ala Asn Ser Thr Trp Glu Pro Glu
        275                 280                 285
Met Pro Gln Cys Ile Lys Asp Ser Lys Pro Thr Asp Pro Pro Ala Thr
    290                 295                 300
Pro Gly Pro Ser His Pro Gly Pro Pro Ser Pro Ser Asp Ala Ser Pro
305                 310                 315                 320
Pro Lys Asp Ala Glu Ser Leu Asp Gly Gly Ile Ile Ala Ala Ile Val
                325                 330                 335
Val Gly Val Leu Ala Ala Ile Ala Val Ile Ala Gly Gly Val Tyr Phe
            340                 345                 350
Phe His His Lys Tyr Asn Lys Lys Arg Ser Lys
        355                 360

<210> 23
<211> 349
<212> PRT
<213> Artificial Sequence

<220>
<223> HS_CD46

<400> 23
Met Glu Pro Pro Gly Arg Arg Glu Cys Pro Phe Pro Ser Trp Arg Phe
1               5                   10                  15
Pro Gly Leu Leu Leu Ala Ala Met Val Leu Leu Leu Tyr Ser Phe Ser
            20                  25                  30
Asp Ala Cys Glu Glu Pro Pro Thr Phe Glu Ala Met Glu Leu Ile Gly
        35                  40                  45
Lys Pro Lys Pro Tyr Tyr Glu Ile Gly Glu Arg Val Asp Tyr Lys Cys
    50                  55                  60
Lys Lys Gly Tyr Phe Tyr Ile Pro Pro Leu Ala Thr His Thr Ile Cys
65                  70                  75                  80
Asp Arg Asn His Thr Trp Leu Pro Val Ser Asp Asp Ala Cys Tyr Arg
                85                  90                  95
Glu Thr Cys Pro Tyr Ile Arg Asp Pro Leu Asn Gly Gln Ala Val Pro
            100                 105                 110
Ala Asn Gly Thr Tyr Glu Phe Gly Tyr Gln Met His Phe Ile Cys Asn
        115                 120                 125
Glu Gly Tyr Tyr Leu Ile Gly Glu Glu Ile Leu Tyr Cys Glu Leu Lys
    130                 135                 140
Gly Ser Val Ala Ile Trp Ser Gly Lys Pro Pro Ile Cys Glu Lys Val
145                 150                 155                 160
Leu Cys Thr Pro Pro Pro Lys Ile Lys Asn Gly Lys His Thr Phe Ser
                165                 170                 175
Glu Val Glu Val Phe Glu Tyr Leu Asp Ala Val Thr Tyr Ser Cys Asp
            180                 185                 190
Pro Ala Pro Gly Pro Asp Pro Phe Ser Leu Ile Gly Glu Ser Thr Ile
        195                 200                 205
Tyr Cys Gly Asp Asn Ser Val Trp Ser Arg Ala Ala Pro Glu Cys Lys
    210                 215                 220
Val Val Lys Cys Arg Phe Pro Val Val Glu Asn Gly Lys Gln Ile Ser
225                 230                 235                 240

Gly Phe Gly Lys Lys Phe Tyr Tyr Lys Ala Thr Val Met Phe Glu Cys
                245                 250                 255
Asp Lys Gly Phe Tyr Leu Asp Gly Ser Asp Thr Ile Val Cys Asp Ser
            260                 265                 270
Asn Ser Thr Trp Asp Pro Pro Val Pro Lys Cys Leu Lys Gly Pro Arg
        275                 280                 285
Pro Thr Tyr Lys Pro Pro Val Ser Asn Tyr Pro Gly Tyr Pro Lys Pro
    290                 295                 300
Glu Glu Gly Ile Leu Asp Ser Leu Asp Val Trp Val Ile Ala Val Ile
305                 310                 315                 320
Val Ile Ala Ile Gly Lys Gln Met Val Glu Leu Asn Met Pro Leu Thr
                325                 330                 335
Arg Leu Asn Gln Pro Leu Gln Gln Ser Arg Glu Ala Glu
            340                 345

<210> 24
<211> 381
<212> PRT
<213> Artificial Sequence

<220>
<223> CD55

<400> 24
Met Thr Val Ala Arg Pro Ser Val Pro Ala Ala Leu Pro Leu Leu Gly
1               5                   10                  15
Glu Leu Pro Arg Leu Leu Leu Leu Val Leu Leu Cys Leu Pro Ala Val
            20                  25                  30
Trp Gly Asp Cys Gly Leu Pro Pro Asp Val Pro Asn Ala Gln Pro Ala
        35                  40                  45
Leu Glu Gly Arg Thr Ser Phe Pro Glu Asp Thr Val Ile Thr Tyr Lys
    50                  55                  60
Cys Glu Glu Ser Phe Val Lys Ile Pro Gly Glu Lys Asp Ser Val Ile
65                  70                  75                  80
Cys Leu Lys Gly Ser Gln Trp Ser Asp Ile Glu Glu Phe Cys Asn Arg
                85                  90                  95
Ser Cys Glu Val Pro Thr Arg Leu Asn Ser Ala Ser Leu Lys Gln Pro
            100                 105                 110
Tyr Ile Thr Gln Asn Tyr Phe Pro Val Gly Thr Val Val Glu Tyr Glu
        115                 120                 125
Cys Arg Pro Gly Tyr Arg Arg Glu Pro Ser Leu Ser Pro Lys Leu Thr
    130                 135                 140
Cys Leu Gln Asn Leu Lys Trp Ser Thr Ala Val Glu Phe Cys Lys Lys
145                 150                 155                 160
Lys Ser Cys Pro Asn Pro Gly Glu Ile Arg Asn Gly Gln Ile Asp Val
                165                 170                 175
Pro Gly Gly Ile Leu Phe Gly Ala Thr Ile Ser Phe Ser Cys Asn Thr
            180                 185                 190
Gly Tyr Lys Leu Phe Gly Ser Thr Ser Ser Phe Cys Leu Ile Ser Gly
        195                 200                 205
Ser Ser Val Gln Trp Ser Asp Pro Leu Pro Glu Cys Arg Glu Ile Tyr
    210                 215                 220
Cys Pro Ala Pro Pro Gln Ile Asp Asn Gly Ile Ile Gln Gly Glu Arg
225                 230                 235                 240
Asp His Tyr Gly Tyr Arg Gln Ser Val Thr Tyr Ala Cys Asn Lys Gly
                245                 250                 255
Phe Thr Met Ile Gly Glu His Ser Ile Tyr Cys Thr Val Asn Asn Asp
            260                 265                 270
Glu Gly Glu Trp Ser Gly Pro Pro Pro Glu Cys Arg Gly Lys Ser Leu
        275                 280                 285
Thr Ser Lys Val Pro Pro Thr Val Gln Lys Pro Thr Thr Val Asn Val
    290                 295                 300

Pro Thr Thr Glu Val Ser Pro Thr Ser Gln Lys Thr Thr Thr Lys Thr
305                 310                 315                 320
Thr Thr Pro Asn Ala Gln Ala Thr Arg Ser Thr Pro Val Ser Arg Thr
                325                 330                 335
Thr Lys His Phe His Glu Thr Thr Pro Asn Lys Gly Ser Gly Thr Thr
            340                 345                 350
Ser Gly Thr Thr Arg Leu Leu Ser Gly His Thr Cys Phe Thr Leu Thr
        355                 360                 365
Gly Leu Leu Gly Thr Leu Val Thr Met Gly Leu Leu Thr
    370                 375                 380

<210> 25
<211> 128
<212> PRT
<213> Artificial Sequence

<220>
<223> CD59

<400> 25
Met Gly Ile Gln Gly Gly Ser Val Leu Phe Gly Leu Leu Leu Val Leu
1               5                   10                  15
Ala Val Phe Cys His Ser Gly His Ser Leu Gln Cys Tyr Asn Cys Pro
            20                  25                  30
Asn Pro Thr Ala Asp Cys Lys Thr Ala Val Asn Cys Ser Ser Asp Phe
        35                  40                  45
Asp Ala Cys Leu Ile Thr Lys Ala Gly Leu Gln Val Tyr Asn Lys Cys
    50                  55                  60
Trp Lys Phe Glu His Cys Asn Phe Asn Asp Val Thr Thr Arg Leu Arg
65                  70                  75                  80
Glu Asn Glu Leu Thr Tyr Tyr Cys Cys Lys Lys Asp Leu Cys Asn Phe
                85                  90                  95
Asn Glu Gln Leu Glu Asn Gly Gly Thr Ser Leu Ser Glu Lys Thr Val
            100                 105                 110
Leu Leu Leu Val Thr Pro Phe Leu Ala Ala Ala Trp Ser Leu His Pro
        115                 120                 125

<210> 26
<211> 360
<212> DNA
<213> Artificial Sequence

<220>
<223> beta2microglobulin encoding sequence of rattus norvegicus

<400> 26
atggctcgct cggtgaccgt gatctttctg gtgcttgtct ctctggccgt cgtgcttgcc      60

attcagaaaa ctccccaaat tcaagtgtac tctcgccatc caccggagaa tgggaagccc     120

aacttcctca actgctacgt gtctcagttc cacccacctc aaatagaaat tgagctactg     180

aagaatggaa agaagatacc aaatatcgag atgtcagatc tgtccttcag caaggactgg     240

tctttctaca tcctggctca cactgaattc acacccaccg agaccgatgt atatgcttgc     300

agagttaaac acgtcactct gaaggagccc aaaaccgtca cctgggaccg agacatgtaa     360

<210> 27
<211> 3159
<212> DNA
<213> Artificial Sequence

<220>
<223> MHC class II transactivator (CIITA) rattus norvegicus encoding
      sequence

<220>
<221> CDS
<222> 1..3159
<223> /note="CIITA"
      /transl_table=1

<400> 27
atg gag ctg ggg tct cca gag ggc agc tac ctg gag ctc ctt aac agt      48
Met Glu Leu Gly Ser Pro Glu Gly Ser Tyr Leu Glu Leu Leu Asn Ser
1               5                   10                  15

gat gct gac ccc cta cac ctc tac cac ctc tat gac cag atg gac ctt      96
Asp Ala Asp Pro Leu His Leu Tyr His Leu Tyr Asp Gln Met Asp Leu
            20                  25                  30

gct ggg gag gaa gag atc gaa ctc aac tca gag aca gac atg gac acc      144
Ala Gly Glu Glu Glu Ile Glu Leu Asn Ser Glu Thr Asp Met Asp Thr
        35                  40                  45

atc aac tgt gac cag ttc agc aag ctg ttg cag gat atg gaa atg gat      192
Ile Asn Cys Asp Gln Phe Ser Lys Leu Leu Gln Asp Met Glu Met Asp
    50                  55                  60

gaa gag acc cgg gag gcc tat gcc aat atc gcg gaa ctg gat cag tac      240
Glu Glu Thr Arg Glu Ala Tyr Ala Asn Ile Ala Glu Leu Asp Gln Tyr
65                  70                  75                  80

gtg ttc cag gat acc cag ctg gag ggc ctg agc aag gac ctc ttc ata      288
Val Phe Gln Asp Thr Gln Leu Glu Gly Leu Ser Lys Asp Leu Phe Ile
                85                  90                  95

cag cac att gga cca gag gaa ggc ttt ggt gag aac gta gag ggc cct      336
Gln His Ile Gly Pro Glu Glu Gly Phe Gly Glu Asn Val Glu Gly Pro
            100                 105                 110

gca gaa tca gga gag aag cct cag aag aga cgc ttc cca gaa gag cat      384
Ala Glu Ser Gly Glu Lys Pro Gln Lys Arg Arg Phe Pro Glu Glu His
        115                 120                 125

gct atg gac tca aag cac agg aaa cta gtg cct ccc tct agg acc tca      432
Ala Met Asp Ser Lys His Arg Lys Leu Val Pro Pro Ser Arg Thr Ser
    130                 135                 140

ctg aac tat ttg gat ctc ccc gct ggg cat atc cag atc ttc acc act      480
Leu Asn Tyr Leu Asp Leu Pro Ala Gly His Ile Gln Ile Phe Thr Thr
145                 150                 155                 160

ctg ccc cag gga ctc tgg caa atc tca ggg gtt ggc aca ggg ctc tcc      528
Leu Pro Gln Gly Leu Trp Gln Ile Ser Gly Val Gly Thr Gly Leu Ser
                165                 170                 175

agt gtc cta gtc tac cac ggt gag atg ccc cag gtc aac caa tta ctc      576
Ser Val Leu Val Tyr His Gly Glu Met Pro Gln Val Asn Gln Leu Leu

            180                 185                 190

tct tca agc agc ccc act gtc ccc agt ctc ccc gag tcc cca gac cgg 624
Ser Ser Ser Ser Pro Thr Val Pro Ser Leu Pro Glu Ser Pro Asp Arg
        195                 200                 205

cct ggc tcc acc agc ccc ttc gcg cca tcc aca gct gac ctg ccc ggc 672
Pro Gly Ser Thr Ser Pro Phe Ala Pro Ser Thr Ala Asp Leu Pro Gly
    210                 215                 220

atg cct gaa cct gct ctg acc tcc cgt gta aat gag aca gag gac aca 720
Met Pro Glu Pro Ala Leu Thr Ser Arg Val Asn Glu Thr Glu Asp Thr
225                 230                 235                 240

aca ccc ccc tcc cca tgc caa gag gat ccc gag cct tcc atc aag ctt 768
Thr Pro Pro Ser Pro Cys Gln Glu Asp Pro Glu Pro Ser Ile Lys Leu
                245                 250                 255

cca aaa tgg cca gag tcc gtg gag cgg ttc cag cac tcc cta cag gac 816
Pro Lys Trp Pro Glu Ser Val Glu Arg Phe Gln His Ser Leu Gln Asp
            260                 265                 270

aag tac caa gca ctg ccc ggg agc cca agt ggt cct ctg gta gcc atg 864
Lys Tyr Gln Ala Leu Pro Gly Ser Pro Ser Gly Pro Leu Val Ala Met
        275                 280                 285

gag ctg gtg cgg gcc agg ttg gaa aga ggt agc aac aag agt cag gaa 912
Glu Leu Val Arg Ala Arg Leu Glu Arg Gly Ser Asn Lys Ser Gln Glu
    290                 295                 300

agg gag ctg gcc act cct gac tgg aca gag cgc cag cta gcc cat ggc 960
Arg Glu Leu Ala Thr Pro Asp Trp Thr Glu Arg Gln Leu Ala His Gly
305                 310                 315                 320

ggt ctg gcc gag gta ctt cgg gtt gtc ggc gac tcc agg aga tca cga 1008
Gly Leu Ala Glu Val Leu Arg Val Val Gly Asp Ser Arg Arg Ser Arg
                325                 330                 335

gag aca cag gtg gtc gct gtg ctg ggc aag gct ggt cag ggc aag agc 1056
Glu Thr Gln Val Val Ala Val Leu Gly Lys Ala Gly Gln Gly Lys Ser
            340                 345                 350

cac tgg gcc agg aca gtg agc cac att tgg gca caa ggc cag tta cca 1104
His Trp Ala Arg Thr Val Ser His Ile Trp Ala Gln Gly Gln Leu Pro
        355                 360                 365

caa tat gac ttt gtc ttc tac gtc ccc tgt caa tgc ttg gat cgc cct 1152
Gln Tyr Asp Phe Val Phe Tyr Val Pro Cys Gln Cys Leu Asp Arg Pro
    370                 375                 380

ggg gac acc tac cac ctg cgg gat ctg ctc tgt ccc ccg aac ctg cag 1200
Gly Asp Thr Tyr His Leu Arg Asp Leu Leu Cys Pro Pro Asn Leu Gln
385                 390                 395                 400

cca ctg gcc atg aat gac gag gtc att gat cat att gtg agg cag ccg 1248
Pro Leu Ala Met Asn Asp Glu Val Ile Asp His Ile Val Arg Gln Pro
                405                 410                 415

gac cgt gtt ctg ctc atc cta gat gct ttt gag gag cta gag gcc caa 1296
Asp Arg Val Leu Leu Ile Leu Asp Ala Phe Glu Glu Leu Glu Ala Gln
            420                 425                 430

gac agc ctc ctg cat gga ccc tgt gga cct ctg tcc cca gag ccc tgc 1344

Asp Ser Leu Leu His Gly Pro Cys Gly Pro Leu Ser Pro Glu Pro Cys
        435                 440                 445

tcc ctc cgg gga ctg ctg gct ggg ctc ttc cag cgt aag ctg ctg cga     1392
Ser Leu Arg Gly Leu Leu Ala Gly Leu Phe Gln Arg Lys Leu Leu Arg
    450                 455                 460

ggc tgc acc ctg ctc ctc aca gcc cgg ccc cgg ggc cgc ctg gct cag     1440
Gly Cys Thr Leu Leu Leu Thr Ala Arg Pro Arg Gly Arg Leu Ala Gln
465                 470                 475                 480

agc ctg agc aag gca gat gcc atc ttc gag gtg ccc agc ttc tct acg     1488
Ser Leu Ser Lys Ala Asp Ala Ile Phe Glu Val Pro Ser Phe Ser Thr
                485                 490                 495

aaa cag gcc cag atc tat atg agg cac tac ttt gag aac tca ggg aca     1536
Lys Gln Ala Gln Ile Tyr Met Arg His Tyr Phe Glu Asn Ser Gly Thr
            500                 505                 510

gca ggg agt cac gac aag gcc ctg ggc ctc ctg gag ggc cag cct ttt     1584
Ala Gly Ser His Asp Lys Ala Leu Gly Leu Leu Glu Gly Gln Pro Phe
        515                 520                 525

ctc ctc acc cac agt cac agc cct gtt ctg tgc agt gcc gtg tgt cag     1632
Leu Leu Thr His Ser His Ser Pro Val Leu Cys Ser Ala Val Cys Gln
    530                 535                 540

ctc tcc aag gcc ctg ctg gat cag ggc gca ggg gcc cag cta cct tgc     1680
Leu Ser Lys Ala Leu Leu Asp Gln Gly Ala Gly Ala Gln Leu Pro Cys
545                 550                 555                 560

aca ctc aca gga ctc tat gtt ggc ctg cta ggc cct gca gct cag aac     1728
Thr Leu Thr Gly Leu Tyr Val Gly Leu Leu Gly Pro Ala Ala Gln Asn
                565                 570                 575

agt cct cct gga gcc tta gtt gag ttg gcc aag cta gcc tgg gag ctg     1776
Ser Pro Pro Gly Ala Leu Val Glu Leu Ala Lys Leu Ala Trp Glu Leu
            580                 585                 590

gga cgg agg cac caa agc acc ttg cag gaa acc cag ttt cca tcc gtg     1824
Gly Arg Arg His Gln Ser Thr Leu Gln Glu Thr Gln Phe Pro Ser Val
        595                 600                 605

gaa gtg aag acc tgg gct gtg gcc caa ggc ttg atg cag cag acc aca     1872
Glu Val Lys Thr Trp Ala Val Ala Gln Gly Leu Met Gln Gln Thr Thr
    610                 615                 620

gag gcc caa ctg gct ttc tcc agt ttt ctg cta cag tgc ttc ctg ggt     1920
Glu Ala Gln Leu Ala Phe Ser Ser Phe Leu Leu Gln Cys Phe Leu Gly
625                 630                 635                 640

gct gtg tgg ctg gca cag tgc aat gag atg aaa gac aag gag ctg cca     1968
Ala Val Trp Leu Ala Gln Cys Asn Glu Met Lys Asp Lys Glu Leu Pro
                645                 650                 655

cag tac ctg gcc ttg acc ccg agg aag aag cga ccc tat gac aac tgg     2016
Gln Tyr Leu Ala Leu Thr Pro Arg Lys Lys Arg Pro Tyr Asp Asn Trp
            660                 665                 670

ctg gag ggc gta cca cgc ttt ctg gct ggc tta gtt ttc cag cct cga     2064
Leu Glu Gly Val Pro Arg Phe Leu Ala Gly Leu Val Phe Gln Pro Arg
        675                 680                 685

gcc cac tgc ctg gga gct ctg gtg gag cct gca atg gct gca gtg gca     2112
Ala His Cys Leu Gly Ala Leu Val Glu Pro Ala Met Ala Ala Val Ala
    690                 695                 700

gat agg aaa cgg aag gtt ctt acc agg tac ctg aag cgc ttg aag ctg     2160
Asp Arg Lys Arg Lys Val Leu Thr Arg Tyr Leu Lys Arg Leu Lys Leu
705                 710                 715                 720

ggg aca ctt cgg gcc ggg agg ttg ctg gag ctg cta cac tgt gcc cac     2208
Gly Thr Leu Arg Ala Gly Arg Leu Leu Glu Leu Leu His Cys Ala His
                725                 730                 735

gaa aca cag gaa cct ggt att tgg aag cat gtt tca cac cag ctc cca     2256
Glu Thr Gln Glu Pro Gly Ile Trp Lys His Val Ser His Gln Leu Pro
            740                 745                 750

ggg cac ctc tcc ttc ctg ggc aca cgg ctc aca gcc cca gat gtg tat     2304
Gly His Leu Ser Phe Leu Gly Thr Arg Leu Thr Ala Pro Asp Val Tyr
        755                 760                 765

gtt ctg ggc agg gcc ttg gag aca gcc agc cag gac ttc tcc ttg gac     2352
Val Leu Gly Arg Ala Leu Glu Thr Ala Ser Gln Asp Phe Ser Leu Asp
    770                 775                 780

ctt cgt cag act ggc atc gag ccc tcc aga ctg gga gac ctc gtg gga     2400
Leu Arg Gln Thr Gly Ile Glu Pro Ser Arg Leu Gly Asp Leu Val Gly
785                 790                 795                 800

ctc agc tgt gtc acc agt ttc agg gcc tcc ttg agt gac acg atg gca     2448
Leu Ser Cys Val Thr Ser Phe Arg Ala Ser Leu Ser Asp Thr Met Ala
                805                 810                 815

tta tgg gag tcc cta cag cat cag gga gaa acc cag cta ctc cag gcg     2496
Leu Trp Glu Ser Leu Gln His Gln Gly Glu Thr Gln Leu Leu Gln Ala
            820                 825                 830

gca gag gag aag ttc acc att gag cca ttt aag gcc aaa tcc cca aag     2544
Ala Glu Glu Lys Phe Thr Ile Glu Pro Phe Lys Ala Lys Ser Pro Lys
        835                 840                 845

gat gtg gaa gac ctg gac agc ctt gtg cag acc cag agg ctg aga aac     2592
Asp Val Glu Asp Leu Asp Ser Leu Val Gln Thr Gln Arg Leu Arg Asn
    850                 855                 860

ccc tca gcg gat gca gcc aag gat ctt cct gcc atc cgg gac ctt aag     2640
Pro Ser Ala Asp Ala Ala Lys Asp Leu Pro Ala Ile Arg Asp Leu Lys
865                 870                 875                 880

aag cta gag ttt gca ttg ggc ccc gtc ttg ggc ccg cag gct ttc ccc     2688
Lys Leu Glu Phe Ala Leu Gly Pro Val Leu Gly Pro Gln Ala Phe Pro
                885                 890                 895

aca ctg gcc aag atc ctt cca gca ttc tct tcc ctg cag cac ctg gac     2736
Thr Leu Ala Lys Ile Leu Pro Ala Phe Ser Ser Leu Gln His Leu Asp
            900                 905                 910

ctg gac tca ctt agc gag aac aag atc ggg gac aag ggt gtg tcg aag     2784
Leu Asp Ser Leu Ser Glu Asn Lys Ile Gly Asp Lys Gly Val Ser Lys
        915                 920                 925

ctc tca gcc acc ttc cct cag ctg aag gcc ctg gag acg ctc aac ctg     2832
Leu Ser Ala Thr Phe Pro Gln Leu Lys Ala Leu Glu Thr Leu Asn Leu
    930                 935                 940

tcc cag aat agc atc acc gat gtg gga gct tgc aag ctt gca gaa gcc     2880
Ser Gln Asn Ser Ile Thr Asp Val Gly Ala Cys Lys Leu Ala Glu Ala
945                 950                 955                 960

ctg ccg gcc ctg gcc aag tcc ctc cta agg ctg agc ttg tac aac aac     2928
Leu Pro Ala Leu Ala Lys Ser Leu Leu Arg Leu Ser Leu Tyr Asn Asn
                965                 970                 975

tgc atc tgt gac gaa ggg gcc aag agc ctg gca cga gtg ctt cca gac     2976
Cys Ile Cys Asp Glu Gly Ala Lys Ser Leu Ala Arg Val Leu Pro Asp
            980                 985                 990

atg gtg tcc ctc cgg gtg atg gat gtc cag ttc aac aag ttc acg gct     3024
Met Val Ser Leu Arg Val Met Asp Val Gln Phe Asn Lys Phe Thr Ala
        995                 1000                1005

gtc ggg gcc cag cag ctg acc tcc agc ctt cag aag tgc cct cag gtg     3072
Val Gly Ala Gln Gln Leu Thr Ser Ser Leu Gln Lys Cys Pro Gln Val
    1010                1015                1020

gag aca ctg gca atg tgg aca ccc act atc ccc ttt ggg gtc cag gaa     3120
Glu Thr Leu Ala Met Trp Thr Pro Thr Ile Pro Phe Gly Val Gln Glu
1025                1030                1035                1040

cac ctg cag cag ctg gat gcc cgg att agc ctg aga tga                 3159
His Leu Gln Gln Leu Asp Ala Arg Ile Ser Leu Arg
                1045                1050

<210> 28
<211> 360
<212> DNA
<213> Artificial Sequence

<220>
<223> Homo sapiens 2m encoding sequence

<220>
<221> CDS
<222> 1..360
<223> /note="human beta2microglobulin"
      /transl_table=1

<400> 28
atg tct cgc tcc gtg gcc tta gct gtg ctc gcg cta ctc tct ctt tct      48
Met Ser Arg Ser Val Ala Leu Ala Val Leu Ala Leu Leu Ser Leu Ser
1               5                   10                  15

ggc ctg gag gct atc cag cgt act cca aag att cag gtt tac tca cgt      96
Gly Leu Glu Ala Ile Gln Arg Thr Pro Lys Ile Gln Val Tyr Ser Arg
            20                  25                  30

cat cca gca gag aat gga aag tca aat ttc ctg aat tgc tat gtg tct     144
His Pro Ala Glu Asn Gly Lys Ser Asn Phe Leu Asn Cys Tyr Val Ser
        35                  40                  45

ggg ttt cat cca tcc gac att gaa gtt gac tta ctg aag aat gga gag     192
Gly Phe His Pro Ser Asp Ile Glu Val Asp Leu Leu Lys Asn Gly Glu
    50                  55                  60

aga att gaa aaa gtg gag cat tca gac ttg tct ttc agc aag gac tgg     240
Arg Ile Glu Lys Val Glu His Ser Asp Leu Ser Phe Ser Lys Asp Trp

65                  70                  75                  80

tct ttc tat ctc ttg tac tac act gaa ttc acc ccc act gaa aaa gat     288
Ser Phe Tyr Leu Leu Tyr Tyr Thr Glu Phe Thr Pro Thr Glu Lys Asp
                85                  90                  95

gag tat gcc tgc cgt gtg aac cat gtg act ttg tca cag ccc aag ata     336
Glu Tyr Ala Cys Arg Val Asn His Val Thr Leu Ser Gln Pro Lys Ile
            100                 105                 110

gtt aag tgg gat cga gac atg taa                                     360
Val Lys Trp Asp Arg Asp Met
        115

<210> 29
<211> 3393
<212> DNA
<213> Artificial Sequence

<220>
<223> Homo sapiens CIITA encoding sequence

<220>
<221> CDS
<222> 1..3393
<223> /note="CIITA"
      /transl_table=1

<400> 29
atg cgt tgc ctg gct cca cgc cct gct ggg tcc tac ctg tca gag ccc      48
Met Arg Cys Leu Ala Pro Arg Pro Ala Gly Ser Tyr Leu Ser Glu Pro
1               5                   10                  15

caa ggc agc tca cag tgt gcc acc atg gag ttg ggg ccc cta gaa ggt      96
Gln Gly Ser Ser Gln Cys Ala Thr Met Glu Leu Gly Pro Leu Glu Gly
            20                  25                  30

ggc tac ctg gag ctt ctt aac agc gat gct gac ccc ctg tgc ctc tac     144
Gly Tyr Leu Glu Leu Leu Asn Ser Asp Ala Asp Pro Leu Cys Leu Tyr
        35                  40                  45

cac ttc tat gac cag atg gac ctg gct gga gaa gaa gag att gag ctc     192
His Phe Tyr Asp Gln Met Asp Leu Ala Gly Glu Glu Glu Ile Glu Leu
    50                  55                  60

tac tca gaa ccc gac aca gac acc atc aac tgc gac cag ttc agc agg     240
Tyr Ser Glu Pro Asp Thr Asp Thr Ile Asn Cys Asp Gln Phe Ser Arg
65                  70                  75                  80

ctg ttg tgt gac atg gaa ggt gat gaa gag acc agg gag gct tat gcc     288
Leu Leu Cys Asp Met Glu Gly Asp Glu Glu Thr Arg Glu Ala Tyr Ala
                85                  90                  95

aat atc gcg gaa ctg gac cag tat gtc ttc cag gac tcc cag ctg gag     336
Asn Ile Ala Glu Leu Asp Gln Tyr Val Phe Gln Asp Ser Gln Leu Glu
            100                 105                 110

ggc ctg agc aag gac att ttc aag cac ata gga cca gat gaa gtg atc     384
Gly Leu Ser Lys Asp Ile Phe Lys His Ile Gly Pro Asp Glu Val Ile
        115                 120                 125

ggt gag agt atg gag atg cca gca gaa gtt ggg cag aaa agt cag aaa     432

Gly Glu Ser Met Glu Met Pro Ala Glu Val Gly Gln Lys Ser Gln Lys
    130                 135                 140

aga ccc ttc cca gag gag ctt ccg gca gac ctg aag cac tgg aag cca      480
Arg Pro Phe Pro Glu Glu Leu Pro Ala Asp Leu Lys His Trp Lys Pro
145                 150                 155                 160

gct gag ccc ccc act gtg gtg act ggc agt ctc cta gtg gga cca gtg      528
Ala Glu Pro Pro Thr Val Val Thr Gly Ser Leu Leu Val Gly Pro Val
                165                 170                 175

agc gac tgc tcc acc ctg ccc tgc ctg cca ctg cct gcg ctg ttc aac      576
Ser Asp Cys Ser Thr Leu Pro Cys Leu Pro Leu Pro Ala Leu Phe Asn
            180                 185                 190

cag gag cca gcc tcc ggc cag atg cgc ctg gag aaa acc gac cag att      624
Gln Glu Pro Ala Ser Gly Gln Met Arg Leu Glu Lys Thr Asp Gln Ile
        195                 200                 205

ccc atg cct ttc tcc agt tcc tcg ttg agc tgc ctg aat ctc cct gag      672
Pro Met Pro Phe Ser Ser Ser Ser Leu Ser Cys Leu Asn Leu Pro Glu
    210                 215                 220

gga ccc atc cag ttt gtc ccc acc atc tcc act ctg ccc cat ggg ctc      720
Gly Pro Ile Gln Phe Val Pro Thr Ile Ser Thr Leu Pro His Gly Leu
225                 230                 235                 240

tgg caa atc tct gag gct gga aca ggg gtc tcc agt ata ttc atc tac      768
Trp Gln Ile Ser Glu Ala Gly Thr Gly Val Ser Ser Ile Phe Ile Tyr
                245                 250                 255

cat ggt gag gtg ccc cag gcc agc caa gta ccc cct ccc agt gga ttc      816
His Gly Glu Val Pro Gln Ala Ser Gln Val Pro Pro Pro Ser Gly Phe
            260                 265                 270

act gtc cac ggc ctc cca aca tct cca gac cgg cca ggc tcc acc agc      864
Thr Val His Gly Leu Pro Thr Ser Pro Asp Arg Pro Gly Ser Thr Ser
        275                 280                 285

ccc ttc gct cca tca gcc act gac ctg ccc agc atg cct gaa cct gcc      912
Pro Phe Ala Pro Ser Ala Thr Asp Leu Pro Ser Met Pro Glu Pro Ala
    290                 295                 300

ctg acc tcc cga gca aac atg aca gag cac aag acg tcc ccc acc caa      960
Leu Thr Ser Arg Ala Asn Met Thr Glu His Lys Thr Ser Pro Thr Gln
305                 310                 315                 320

tgc ccg gca gct gga gag gtc tcc aac aag ctt cca aaa tgg cct gag     1008
Cys Pro Ala Ala Gly Glu Val Ser Asn Lys Leu Pro Lys Trp Pro Glu
                325                 330                 335

ccg gtg gag cag ttc tac cgc tca ctg cag gac acg tat ggt gcc gag     1056
Pro Val Glu Gln Phe Tyr Arg Ser Leu Gln Asp Thr Tyr Gly Ala Glu
            340                 345                 350

ccc gca ggc ccg gat ggc atc cta gtg gag gtg gat ctg gtg cag gcc     1104
Pro Ala Gly Pro Asp Gly Ile Leu Val Glu Val Asp Leu Val Gln Ala
        355                 360                 365

agg ctg gag agg agc agc agc aag agc ctg gag cgg gaa ctg gcc acc     1152
Arg Leu Glu Arg Ser Ser Ser Lys Ser Leu Glu Arg Glu Leu Ala Thr
    370                 375                 380

ccg gac tgg gca gaa cgg cag ctg gcc caa gga ggc ctg gct gag gtg     1200
Pro Asp Trp Ala Glu Arg Gln Leu Ala Gln Gly Gly Leu Ala Glu Val
385                 390                 395                 400

ctg ttg gct gcc aag gag cac cgg cgg ccg cgt gag aca cga gtg att     1248
Leu Leu Ala Ala Lys Glu His Arg Arg Pro Arg Glu Thr Arg Val Ile
                405                 410                 415

gct gtg ctg ggc aaa gct ggt cag ggc aag agc tat tgg gct ggg gca     1296
Ala Val Leu Gly Lys Ala Gly Gln Gly Lys Ser Tyr Trp Ala Gly Ala
            420                 425                 430

gtg agc cgg gcc tgg gct tgt ggc cgg ctt ccc cag tac gac ttt gtc     1344
Val Ser Arg Ala Trp Ala Cys Gly Arg Leu Pro Gln Tyr Asp Phe Val
        435                 440                 445

ttc tct gtc ccc tgc cat tgc ttg aac cgt ccg ggg gat gcc tat ggc     1392
Phe Ser Val Pro Cys His Cys Leu Asn Arg Pro Gly Asp Ala Tyr Gly
    450                 455                 460

ctg cag gat ctg ctc ttc tcc ctg ggc cca cag cca ctc gtg gcg gcc     1440
Leu Gln Asp Leu Leu Phe Ser Leu Gly Pro Gln Pro Leu Val Ala Ala
465                 470                 475                 480

gat gag gtt ttc agc cac atc ttg aag aga cct gac cgc gtt ctg ctc     1488
Asp Glu Val Phe Ser His Ile Leu Lys Arg Pro Asp Arg Val Leu Leu
                485                 490                 495

atc cta gac ggc ttc gag gag ctg gaa gcg caa gat ggc ttc ctg cac     1536
Ile Leu Asp Gly Phe Glu Glu Leu Glu Ala Gln Asp Gly Phe Leu His
            500                 505                 510

agc acg tgc gga ccg gca ccg gcg gag ccc tgc tcc ctc cgg ggg ctg     1584
Ser Thr Cys Gly Pro Ala Pro Ala Glu Pro Cys Ser Leu Arg Gly Leu
        515                 520                 525

ctg gcc ggc ctt ttc cag aag aag ctg ctc cga ggt tgc acc ctc ctc     1632
Leu Ala Gly Leu Phe Gln Lys Lys Leu Leu Arg Gly Cys Thr Leu Leu
    530                 535                 540

ctc aca gcc cgg ccc cgg ggc cgc ctg gtc cag agc ctg agc aag gcc     1680
Leu Thr Ala Arg Pro Arg Gly Arg Leu Val Gln Ser Leu Ser Lys Ala
545                 550                 555                 560

gac gcc cta ttt gag ctg tcc ggc ttc tcc atg gag cag gcc cag gca     1728
Asp Ala Leu Phe Glu Leu Ser Gly Phe Ser Met Glu Gln Ala Gln Ala
                565                 570                 575

tac gtg atg cgc tac ttt gag agc tca ggg atg aca gag cac caa gac     1776
Tyr Val Met Arg Tyr Phe Glu Ser Ser Gly Met Thr Glu His Gln Asp
            580                 585                 590

aga gcc ctg acg ctc ctc cgg gac cgg cca ctt ctt ctc agt cac agc     1824
Arg Ala Leu Thr Leu Leu Arg Asp Arg Pro Leu Leu Leu Ser His Ser
        595                 600                 605

cac agc cct act ttg tgc cgg gca gtg tgc cag ctc tca gag gcc ctg     1872
His Ser Pro Thr Leu Cys Arg Ala Val Cys Gln Leu Ser Glu Ala Leu
    610                 615                 620

ctg gag ctt ggg gag gac gcc aag ctg ccc tcc acg ctc acg gga ctc     1920
Leu Glu Leu Gly Glu Asp Ala Lys Leu Pro Ser Thr Leu Thr Gly Leu
625                 630                 635                 640

tat gtc ggc ctg ctg ggc cgt gca gcc ctc gac agc ccc ccc ggg gcc     1968
Tyr Val Gly Leu Leu Gly Arg Ala Ala Leu Asp Ser Pro Pro Gly Ala
                645                 650                 655

ctg gca gag ctg gcc aag ctg gcc tgg gag ctg ggc cgc aga cat caa     2016
Leu Ala Glu Leu Ala Lys Leu Ala Trp Glu Leu Gly Arg Arg His Gln
            660                 665                 670

agt acc cta cag gag gac cag ttc cca tcc gca gac gtg agg acc tgg     2064
Ser Thr Leu Gln Glu Asp Gln Phe Pro Ser Ala Asp Val Arg Thr Trp
        675                 680                 685

gcg atg gcc aaa ggc tta gtc caa cac cca ccg cgg gcc gca gag tcc     2112
Ala Met Ala Lys Gly Leu Val Gln His Pro Pro Arg Ala Ala Glu Ser
    690                 695                 700

gag ctg gcc ttc ccc agc ttc ctc ctg caa tgc ttc ctg ggg gcc ctg     2160
Glu Leu Ala Phe Pro Ser Phe Leu Leu Gln Cys Phe Leu Gly Ala Leu
705                 710                 715                 720

tgg ctg gct ctg agt ggc gaa atc aag gac aag gag ctc ccg cag tac     2208
Trp Leu Ala Leu Ser Gly Glu Ile Lys Asp Lys Glu Leu Pro Gln Tyr
                725                 730                 735

cta gca ttg acc cca agg aag aag agg ccc tat gac aac tgg ctg gag     2256
Leu Ala Leu Thr Pro Arg Lys Lys Arg Pro Tyr Asp Asn Trp Leu Glu
            740                 745                 750

ggc gtg cca cgc ttt ctg gct ggg ctg atc ttc cag cct ccc gcc cgc     2304
Gly Val Pro Arg Phe Leu Ala Gly Leu Ile Phe Gln Pro Pro Ala Arg
        755                 760                 765

tgc ctg gga gcc cta ctc ggg cca tcg gcg gct gcc tcg gtg gac agg     2352
Cys Leu Gly Ala Leu Leu Gly Pro Ser Ala Ala Ala Ser Val Asp Arg
    770                 775                 780

aag cag aag gtg ctt gcg agg tac ctg aag cgg ctg cag ccg ggg aca     2400
Lys Gln Lys Val Leu Ala Arg Tyr Leu Lys Arg Leu Gln Pro Gly Thr
785                 790                 795                 800

ctg cgg gcg cgg cag ctg ctg gag ctg ctg cac tgc gcc cac gag gcc     2448
Leu Arg Ala Arg Gln Leu Leu Glu Leu Leu His Cys Ala His Glu Ala
                805                 810                 815

gag gag gct gga att tgg cag cac gtg gta cag gag ctc ccc ggc cgc     2496
Glu Glu Ala Gly Ile Trp Gln His Val Val Gln Glu Leu Pro Gly Arg
            820                 825                 830

ctc tct ttt ctg ggc acc cgc ctc acg cct cct gat gca cat gta ctg     2544
Leu Ser Phe Leu Gly Thr Arg Leu Thr Pro Pro Asp Ala His Val Leu
        835                 840                 845

ggc aag gcc ttg gag gcg gcg ggc caa gac ttc tcc ctg gac ctc cgc     2592
Gly Lys Ala Leu Glu Ala Ala Gly Gln Asp Phe Ser Leu Asp Leu Arg
    850                 855                 860

agc act ggc att tgc ccc tct gga ttg ggg agc ctc gtg gga ctc agc     2640
Ser Thr Gly Ile Cys Pro Ser Gly Leu Gly Ser Leu Val Gly Leu Ser
865                 870                 875                 880

tgt gtc acc cgt ttc agg gct gcc ttg agc gac acg gtg gcg ctg tgg     2688
Cys Val Thr Arg Phe Arg Ala Ala Leu Ser Asp Thr Val Ala Leu Trp

885                 890                 895

gag tcc ctg cag cag cat ggg gag acc aag cta ctt cag gca gca gag     2736
Glu Ser Leu Gln Gln His Gly Glu Thr Lys Leu Leu Gln Ala Ala Glu
            900                 905                 910

gag aag ttc acc atc gag cct ttc aaa gcc aag tcc ctg aag gat gtg     2784
Glu Lys Phe Thr Ile Glu Pro Phe Lys Ala Lys Ser Leu Lys Asp Val
        915                 920                 925

gaa gac ctg gga aag ctt gtg cag act cag agg acg aga agt tcc tcg     2832
Glu Asp Leu Gly Lys Leu Val Gln Thr Gln Arg Thr Arg Ser Ser Ser
    930                 935                 940

gaa gac aca gct ggg gag ctc cct gct gtt cgg gac cta aag aaa ctg     2880
Glu Asp Thr Ala Gly Glu Leu Pro Ala Val Arg Asp Leu Lys Lys Leu
945                 950                 955                 960

gag ttt gcg ctg ggc cct gtc tca ggc ccc cag gct ttc ccc aaa ctg     2928
Glu Phe Ala Leu Gly Pro Val Ser Gly Pro Gln Ala Phe Pro Lys Leu
                965                 970                 975

gtg cgg atc ctc acg gcc ttt tcc tcc ctg cag cat ctg gac ctg gat     2976
Val Arg Ile Leu Thr Ala Phe Ser Ser Leu Gln His Leu Asp Leu Asp
            980                 985                 990

gcg ctg agt gag aac aag atc ggg gac gag ggt gtc tcg cag ctc tca     3024
Ala Leu Ser Glu Asn Lys Ile Gly Asp Glu Gly Val Ser Gln Leu Ser
        995                 1000                1005

gcc acc ttc ccc cag ctg aag tcc ttg gaa acc ctc aat ctg tcc cag     3072
Ala Thr Phe Pro Gln Leu Lys Ser Leu Glu Thr Leu Asn Leu Ser Gln
    1010                1015                1020

aac aac atc act gac ctg ggt gcc tac aaa ctc gcc gag gcc ctg cct     3120
Asn Asn Ile Thr Asp Leu Gly Ala Tyr Lys Leu Ala Glu Ala Leu Pro
1025                1030                1035                1040

tcg ctc gct gca tcc ctg ctc agg cta agc ttg tac aat aac tgc atc     3168
Ser Leu Ala Ala Ser Leu Leu Arg Leu Ser Leu Tyr Asn Asn Cys Ile
                1045                1050                1055

tgc gac gtg gga gcc gag agc ttg gct cgt gtg ctt ccg gac atg gtg     3216
Cys Asp Val Gly Ala Glu Ser Leu Ala Arg Val Leu Pro Asp Met Val
            1060                1065                1070

tcc ctc cgg gtg atg gac gtc cag tac aac aag ttc acg gct gcc ggg     3264
Ser Leu Arg Val Met Asp Val Gln Tyr Asn Lys Phe Thr Ala Ala Gly
        1075                1080                1085

gcc cag cag ctc gct gcc agc ctt cgg agg tgt cct cat gtg gag acg     3312
Ala Gln Gln Leu Ala Ala Ser Leu Arg Arg Cys Pro His Val Glu Thr
    1090                1095                1100

ctg gcg atg tgg acg ccc acc atc cca ttc agt gtc cag gaa cac ctg     3360
Leu Ala Met Trp Thr Pro Thr Ile Pro Phe Ser Val Gln Glu His Leu
1105                1110                1115                1120

caa caa cag gat tca cgg atc agc ctg aga tga                         3393
Gln Gln Gln Asp Ser Arg Ile Ser Leu Arg
                1125                1130

<210> 30

<211> 357
<212> DNA
<213> Artificial Sequence

<220>
<223> Sus scrofa 2-microglobulin encoding sequence

<220>
<221> CDS
<222> 1..357
<223> /note="swine beta2microglobulin"
      /transl_table=1

<400> 30
atg gct ccc ctc gtg gcc ttg gtc ctg ctc ggg ctg ctc tca ctg tct      48
Met Ala Pro Leu Val Ala Leu Val Leu Leu Gly Leu Leu Ser Leu Ser
1               5                   10                  15

ggc ctg gat gcg gtc gcg cgt ccc ccg aag gtt cag gtt tac tca cgc      96
Gly Leu Asp Ala Val Ala Arg Pro Pro Lys Val Gln Val Tyr Ser Arg
            20                  25                  30

cac cca gcg gaa aac gga aag cca aat tac ctg aac tgc tat gta tct     144
His Pro Ala Glu Asn Gly Lys Pro Asn Tyr Leu Asn Cys Tyr Val Ser
        35                  40                  45

ggg ttc cat ccg ccc cag att gaa att gat ttg ctg aaa aac ggg gag     192
Gly Phe His Pro Pro Gln Ile Glu Ile Asp Leu Leu Lys Asn Gly Glu
    50                  55                  60

aag atg aac gcg gag cag tca gac ctg tct ttc agc aag gac tgg tct     240
Lys Met Asn Ala Glu Gln Ser Asp Leu Ser Phe Ser Lys Asp Trp Ser
65                  70                  75                  80

ttc tac ctt ctg gtc cac act gag ttc act cct aac gct gtg gat cag     288
Phe Tyr Leu Leu Val His Thr Glu Phe Thr Pro Asn Ala Val Asp Gln
                85                  90                  95

tat agc tgc cgc gtg aag cac gtg act ctc gat aag ccc aag ata gtt     336
Tyr Ser Cys Arg Val Lys His Val Thr Leu Asp Lys Pro Lys Ile Val
            100                 105                 110

aag tgg gat cga gac cac taa                                         357
Lys Trp Asp Arg Asp His
        115

<210> 31
<211> 2697
<212> DNA
<213> Artificial Sequence

<220>
<223> SS_CIITA

<220>
<221> CDS
<222> 1..2697
<223> /note="SS_CIITA"
      /transl_table=1

<400> 31

atg gag ttg ggg cct ctg gag ggt ggg tac ttg gag ctt ctc aac agc 48
Met Glu Leu Gly Pro Leu Glu Gly Gly Tyr Leu Glu Leu Leu Asn Ser
1               5                   10                  15

agt gcc gac cct ctg cag ctc tac cac ctc tat gac cgg atg gac ctg 96
Ser Ala Asp Pro Leu Gln Leu Tyr His Leu Tyr Asp Arg Met Asp Leu
            20                  25                  30

gct gga gaa gaa gag atc gag ctc tgc tca gaa cct gac acg gac acc 144
Ala Gly Glu Glu Glu Ile Glu Leu Cys Ser Glu Pro Asp Thr Asp Thr
        35                  40                  45

atc aac tgc gaa cag ttc agc agg ctg ttg tgc gac atg gaa gca gat 192
Ile Asn Cys Glu Gln Phe Ser Arg Leu Leu Cys Asp Met Glu Ala Asp
    50                  55                  60

gaa gaa acc agg gaa act tac gcc agt atc gcg gaa ctg gac cag tat 240
Glu Glu Thr Arg Glu Thr Tyr Ala Ser Ile Ala Glu Leu Asp Gln Tyr
65                  70                  75                  80

gtt ttt caa gac tct cag ctg gag ggc ctg ggc aaa gac att ttc att 288
Val Phe Gln Asp Ser Gln Leu Glu Gly Leu Gly Lys Asp Ile Phe Ile
                85                  90                  95

gag cac ata gga ttg gaa gaa atg atc agt gag agc gtg gag gtg ctg 336
Glu His Ile Gly Leu Glu Glu Met Ile Ser Glu Ser Val Glu Val Leu
            100                 105                 110

gag gac tca ggg cgg aaa agt cag aaa aga tct ttc ccg gag gag ctg 384
Glu Asp Ser Gly Arg Lys Ser Gln Lys Arg Ser Phe Pro Glu Glu Leu
        115                 120                 125

cct gcg gat ctg aag cac agg aag cta gcc gag ccc ctc gcc atg ccc 432
Pro Ala Asp Leu Lys His Arg Lys Leu Ala Glu Pro Leu Ala Met Pro
    130                 135                 140

atg gtg act ggc act ttc ctg gtg ggg cca gtg agc gac tcc tca gct 480
Met Val Thr Gly Thr Phe Leu Val Gly Pro Val Ser Asp Ser Ser Ala
145                 150                 155                 160

cga ccc tgc cca tca cct cct gct ctg ttc aac aag gaa tca aca ccc 528
Arg Pro Cys Pro Ser Pro Pro Ala Leu Phe Asn Lys Glu Ser Thr Pro
                165                 170                 175

agc cag gcc cag ctg gag gac gct gtc cca atg ccg gcg ccc cct tca 576
Ser Gln Ala Gln Leu Glu Asp Ala Val Pro Met Pro Ala Pro Pro Ser
            180                 185                 190

ggt tcc ttg ttg agc tgc ctg agt gtc cct gct gga cct att cag atc 624
Gly Ser Leu Leu Ser Cys Leu Ser Val Pro Ala Gly Pro Ile Gln Ile
        195                 200                 205

atc ccc acg ctc tcc acc ctg ccc cag ggg ctc tgg cac atc tca ggg 672
Ile Pro Thr Leu Ser Thr Leu Pro Gln Gly Leu Trp His Ile Ser Gly
    210                 215                 220

gcc ggg aca ggg gtc tcc agt ata ctc atc tac caa ggt gag cgt ggg 720
Ala Gly Thr Gly Val Ser Ser Ile Leu Ile Tyr Gln Gly Glu Arg Gly
225                 230                 235                 240

aag cca ggc tcc cca ccc cct ctg cct gtg acc ccg gtc ctg cca cta 768
Lys Pro Gly Ser Pro Pro Pro Leu Pro Val Thr Pro Val Leu Pro Leu
                245                 250                 255

gca tcc tcc cct ccc cca ctc ccg cct ttg acc tgt gca gag act gtg     816
Ala Ser Ser Pro Pro Pro Leu Pro Pro Leu Thr Cys Ala Glu Thr Val
            260                 265                 270

gag cag ttc cac cac tca ctc cgg gac agg tac caa gcc aag ccc gca     864
Glu Gln Phe His His Ser Leu Arg Asp Arg Tyr Gln Ala Lys Pro Ala
        275                 280                 285

ggc ccg gaa ggc atc ctg gtg gag gtg gac ctg gtg agg gtg cgg ctg     912
Gly Pro Glu Gly Ile Leu Val Glu Val Asp Leu Val Arg Val Arg Leu
    290                 295                 300

gag agg agc agc agc aag agt cag gag aga gag ctg gcc tcc ctg gac     960
Glu Arg Ser Ser Ser Lys Ser Gln Glu Arg Glu Leu Ala Ser Leu Asp
305                 310                 315                 320

tgg gca gag cgg cag cca gcc cga ggg ggt ctg gcg gag gtg ctg ctg     1008
Trp Ala Glu Arg Gln Pro Ala Arg Gly Gly Leu Ala Glu Val Leu Leu
                325                 330                 335

gcc gct agc gac cgc cag ggg cca cgc gag acg cag gtg atc gcc gtg     1056
Ala Ala Ser Asp Arg Gln Gly Pro Arg Glu Thr Gln Val Ile Ala Val
            340                 345                 350

ctc ggc aaa gca gga caa ggg aag agt cac tgg gcc cag gcc gtg agc     1104
Leu Gly Lys Ala Gly Gln Gly Lys Ser His Trp Ala Gln Ala Val Ser
        355                 360                 365

tgg gcc tgg gct gac ggc cag ctg cca cag tac gac ttt gtc ttc tgc     1152
Trp Ala Trp Ala Asp Gly Gln Leu Pro Gln Tyr Asp Phe Val Phe Cys
    370                 375                 380

atc ccc tgc cac tgt ttg gac cgg ccg ggg aac acc tac cgc ctg cag     1200
Ile Pro Cys His Cys Leu Asp Arg Pro Gly Asn Thr Tyr Arg Leu Gln
385                 390                 395                 400

gat ctg ctc ttc tcc ctg ggc cca cag ccc ctg ccc atg gac gac gag     1248
Asp Leu Leu Phe Ser Leu Gly Pro Gln Pro Leu Pro Met Asp Asp Glu
                405                 410                 415

gtc ttc agt tac atc ttg agg cgg ccg gac cgc gtt ctg ctc atc ctg     1296
Val Phe Ser Tyr Ile Leu Arg Arg Pro Asp Arg Val Leu Leu Ile Leu
            420                 425                 430

gat gcc ttc gag gag cgc gaa gcc cag gac ggc ttc gtg cac agc gcg     1344
Asp Ala Phe Glu Glu Arg Glu Ala Gln Asp Gly Phe Val His Ser Ala
        435                 440                 445

ggc gga ccc ctg tcc tca gaa ccc cgc tcc ctt cgg ggg ctg ctg gct     1392
Gly Gly Pro Leu Ser Ser Glu Pro Arg Ser Leu Arg Gly Leu Leu Ala
    450                 455                 460

ggg ctc ctc cag cgc aag ctg ctg cga ggc tgc acc ctg ctg ctc acg     1440
Gly Leu Leu Gln Arg Lys Leu Leu Arg Gly Cys Thr Leu Leu Leu Thr
465                 470                 475                 480

gcc cgg ccc cgg ggc cgc ctg gcc cag agc ctg agc aag gcc gac gcc     1488
Ala Arg Pro Arg Gly Arg Leu Ala Gln Ser Leu Ser Lys Ala Asp Ala
                485                 490                 495

ctg ttt gag gtg gcc ggc ttc tcc gca cag cag gcc aag acc tac atg     1536
Leu Phe Glu Val Ala Gly Phe Ser Ala Gln Gln Ala Lys Thr Tyr Met

500                 505                 510

ctg cgc tac ttt gag tgt cgg ggg gcc cgt gag cgc cag aag aga gcc     1584
Leu Arg Tyr Phe Glu Cys Arg Gly Ala Arg Glu Arg Gln Lys Arg Ala
        515                 520                 525

ctg gag ctc ctc cag gca cag ccg ttt ctc ctg agt cac agc cac agc     1632
Leu Glu Leu Leu Gln Ala Gln Pro Phe Leu Leu Ser His Ser His Ser
    530                 535                 540

cct tcc gtg tgc cgg gcc gtg tgc cgg ctc tca gag acc ctc ctg gag     1680
Pro Ser Val Cys Arg Ala Val Cys Arg Leu Ser Glu Thr Leu Leu Glu
545                 550                 555                 560

ctg ggc gag gag gca gag ctg ccc tcc acg ctc acc ggc ctc tac gtc     1728
Leu Gly Glu Glu Ala Glu Leu Pro Ser Thr Leu Thr Gly Leu Tyr Val
                565                 570                 575

ggc ctc cta gga cca gcg gcc cgc gaa agc ccc ccg ggt gcc ctg gtg     1776
Gly Leu Leu Gly Pro Ala Ala Arg Glu Ser Pro Pro Gly Ala Leu Val
            580                 585                 590

gga ctg gcc aga ctg gcc tgg gaa ctg ggc cgc cgt cac cac agc agc     1824
Gly Leu Ala Arg Leu Ala Trp Glu Leu Gly Arg Arg His His Ser Ser
        595                 600                 605

ttg cag gag ggc cag ttc cca tcg gca gag gcc agg gcc tgg gct gtg     1872
Leu Gln Glu Gly Gln Phe Pro Ser Ala Glu Ala Arg Ala Trp Ala Val
    610                 615                 620

gcc caa ggc ttg gtg cag cgt gcc ccg ggg gcc ccg ggg gcc cct gag     1920
Ala Gln Gly Leu Val Gln Arg Ala Pro Gly Ala Pro Gly Ala Pro Glu
625                 630                 635                 640

ctg gcc ttc tcc agc ttc ctc ctg cag tgc ttc ctg ggg gcc gtg tgg     1968
Leu Ala Phe Ser Ser Phe Leu Leu Gln Cys Phe Leu Gly Ala Val Trp
                645                 650                 655

ctg gct ctg agc agc gag atc aag gac aag gag ctg ccg cag tat ttg     2016
Leu Ala Leu Ser Ser Glu Ile Lys Asp Lys Glu Leu Pro Gln Tyr Leu
            660                 665                 670

gca tta acc cct agg aag aag agg ccc tat gac aac tgg ctg gag gct     2064
Ala Leu Thr Pro Arg Lys Lys Arg Pro Tyr Asp Asn Trp Leu Glu Ala
        675                 680                 685

gtg cca cgc ttt ctg gtc ggg ctg gtc ttc cag cct cgc gcc cgc tgc     2112
Val Pro Arg Phe Leu Val Gly Leu Val Phe Gln Pro Arg Ala Arg Cys
    690                 695                 700

ctg gga gcc ctg gca ggg ctg gtg gca gcc acc ttg gcg gac cgg aag     2160
Leu Gly Ala Leu Ala Gly Leu Val Ala Ala Thr Leu Ala Asp Arg Lys
705                 710                 715                 720

cag aag gtg ctc aac agg tac ctg aag cgg ctg cag ccc ggg acc ctg     2208
Gln Lys Val Leu Asn Arg Tyr Leu Lys Arg Leu Gln Pro Gly Thr Leu
                725                 730                 735

cag gca ggg cgg ctg ctg gag ctg ctg cac tgc acg cac gag gcc ctg     2256
Gln Ala Gly Arg Leu Leu Glu Leu Leu His Cys Thr His Glu Ala Leu
            740                 745                 750

gat tct ggg ctt tgg cag cat gtg ctg cag ggg ctc ccg acc caa ctc     2304

Asp Ser Gly Leu Trp Gln His Val Leu Gln Gly Leu Pro Thr Gln Leu
        755                 760                 765

tcc ttt ctg ggc act cgg ctc acg cct ccg gac acc cac gtg ctg ggc     2352
Ser Phe Leu Gly Thr Arg Leu Thr Pro Pro Asp Thr His Val Leu Gly
    770                 775                 780

agc gcc ttg gtg gct gca ggc cga gac ttc tcc ctg gac ctc cgc agc     2400
Ser Ala Leu Val Ala Ala Gly Arg Asp Phe Ser Leu Asp Leu Arg Ser
785                 790                 795                 800

act ggc att gac ccc tct gga ctg ggg agc ctc gtg gga ctc agc tgt     2448
Thr Gly Ile Asp Pro Ser Gly Leu Gly Ser Leu Val Gly Leu Ser Cys
                805                 810                 815

gtc acc cat ttc agg gcc gcc ttg agt gac aca gtg ggg ctg tgg gag     2496
Val Thr His Phe Arg Ala Ala Leu Ser Asp Thr Val Gly Leu Trp Glu
            820                 825                 830

tct cta cag caa cgt ggg gag acc aag cta ctc cag gca ctg gag gag     2544
Ser Leu Gln Gln Arg Gly Glu Thr Lys Leu Leu Gln Ala Leu Glu Glu
        835                 840                 845

aaa ttt acc att gag cct ttc aag gcc aag tcc atg aag gat gtg gaa     2592
Lys Phe Thr Ile Glu Pro Phe Lys Ala Lys Ser Met Lys Asp Val Glu
    850                 855                 860

gac ctg ggc aac ctc gtg cag atc cag agg acg aga agc tct tct gaa     2640
Asp Leu Gly Asn Leu Val Gln Ile Gln Arg Thr Arg Ser Ser Ser Glu
865                 870                 875                 880

gac atg gct ggg gaa ctc cct gct gtc cgg gac cta aag aag ttg gaa     2688
Asp Met Ala Gly Glu Leu Pro Ala Val Arg Asp Leu Lys Lys Leu Glu
                885                 890                 895

ttt gcg tga                                                         2697
Phe Ala


<210> 32
<211> 21
<212> PRT
<213> Artificial Sequence


<220>
<223> [CDS]:1..63 from SEQ ID NO 16

<400> 32
Gly Ser Gly Glu Gly Arg Gly Ser Leu Leu Thr Cys Gly Asp Val Glu
1               5                   10                  15
Glu Asn Pro Gly Pro
            20

<210> 33
<211> 466
<212> PRT
<213> Artificial Sequence


<220>
<223> [CDS]:1..1401 from SEQ ID NO 18

<400> 33
Met Ile Arg Thr Arg Ala Pro Gly Thr Arg Ala Pro Pro Pro Pro Pro
1               5                   10                  15
Leu Leu Leu Ser Leu Ser Leu Leu Leu Leu Leu Leu Ser Pro Thr Val
            20                  25                  30
Ser Gly Asp Cys Gly Pro Pro Pro Asp Ile Pro Asn Ala Thr Pro Asn
        35                  40                  45
Ser Gly Lys His Thr Lys Phe Ala His Gln Ser Gln Val Thr Tyr Ser
    50                  55                  60
Cys Asn Lys Gly Phe Lys Gln Ile Pro Asp Lys Pro Asn Thr Val Val
65                  70                  75                  80
Cys Leu Glu Asn Asp Gln Trp Ser Asn Tyr Glu Thr Phe Cys Asn Lys
                85                  90                  95
Thr Cys Ser Ala Pro Leu Arg Leu Asn Tyr Ala Ser Leu Lys Lys Glu
            100                 105                 110
Tyr Arg Asn Met Asn Phe Phe Pro Ile Gly Thr Thr Val Glu Phe Glu
        115                 120                 125
Cys Arg Pro Gly Phe Gln Lys Val Thr Ser Arg Thr Gly Lys Ser Thr
    130                 135                 140
Cys Leu Glu Glu Leu Val Trp Ser Pro Val Val Glu Phe Cys Lys Lys
145                 150                 155                 160
Arg Ala Cys Pro Asn Pro Arg Glu Leu Lys Asn Gly His Ile Asn Ile
                165                 170                 175
Pro Thr Asp Ile Leu Phe Gly Ser Glu Ile Ile Phe Ser Cys Asp Lys
            180                 185                 190
Gly Tyr Lys Leu Val Gly Val Thr Ser Thr Phe Cys Ser Ile Thr Gly
        195                 200                 205
Asn Ala Val Asp Trp Asp Asp Val Phe Pro Val Cys Thr Thr Ile Phe
    210                 215                 220
Cys Pro Asp Pro Pro Lys Ile Asn Asn Gly Arg Ile Arg Glu Glu Ser
225                 230                 235                 240
Asp Ser Tyr Ala Tyr Arg Gln Ser Val Thr Tyr Ser Cys Asp Gln Gly
                245                 250                 255
Phe Ile Leu Val Gly Asn Ser Ser Val His Cys Thr Leu Asn Gly Glu
            260                 265                 270
Val Gly Glu Trp Ser Ser Ala Pro Pro Lys Cys Ile Glu Arg His Lys
        275                 280                 285
Val Pro Thr Lys Thr Pro Pro Glu Val Asp Ile Pro Ser Thr Met Pro
    290                 295                 300
His Glu Ser Thr Leu Ile Asn Phe Pro Ser Thr Arg Ala Pro Leu Ser
305                 310                 315                 320
His Lys Pro Thr Thr Val Lys Val Pro Ala Thr Gln His Val Pro Val
                325                 330                 335
Ser Lys Thr Thr Val Arg His Pro Thr Arg Thr Ser Lys Asp Arg Gly
            340                 345                 350
Glu Ser Asn Ser Gly Gly Asp His Phe Ile Tyr Gly Lys Phe Asp Pro
        355                 360                 365
Gln Gly Val Lys Arg Asn Cys His His Cys Gly Arg His Ser Pro Tyr
    370                 375                 380
Phe Trp Lys Arg Ile Phe Ser Tyr Asn Cys Leu Arg Lys Thr Ile Lys
385                 390                 395                 400
Met Gln Cys Leu Trp Val Pro Lys Gly Arg Trp Val Lys Pro Phe Asn
                405                 410                 415
Arg Leu Val Phe Trp Thr Pro Leu Gly Ile Gln His Gly Pro Ser Tyr
            420                 425                 430
Pro Gln Gln Cys Ser Cys Ser Lys Ala Val Leu Gly Gln Leu Arg Val
        435                 440                 445
Ile Trp Pro Ile Ser Gln Val Ala Leu Ser Ala Arg Cys Val Ser Val
    450                 455                 460
Ser Phe
465

<210> 34

<211> 1052
<212> PRT
<213> Artificial Sequence

<220>
<223> [CDS]:1..3159 from SEQ ID NO 27

<400> 34
   Met Glu Leu Gly Ser Pro Glu Gly Ser Tyr Leu Glu Leu Leu Asn Ser
   1               5                   10                  15
   Asp Ala Asp Pro Leu His Leu Tyr His Leu Tyr Asp Gln Met Asp Leu
               20                  25                  30
   Ala Gly Glu Glu Glu Ile Glu Leu Asn Ser Glu Thr Asp Met Asp Thr
           35                  40                  45
   Ile Asn Cys Asp Gln Phe Ser Lys Leu Leu Gln Asp Met Glu Met Asp
       50                  55                  60
   Glu Glu Thr Arg Glu Ala Tyr Ala Asn Ile Ala Glu Leu Asp Gln Tyr
   65                  70                  75                  80
   Val Phe Gln Asp Thr Gln Leu Glu Gly Leu Ser Lys Asp Leu Phe Ile
                   85                  90                  95
   Gln His Ile Gly Pro Glu Glu Gly Phe Gly Glu Asn Val Glu Gly Pro
               100                 105                 110
   Ala Glu Ser Gly Glu Lys Pro Gln Lys Arg Arg Phe Pro Glu Glu His
           115                 120                 125
   Ala Met Asp Ser Lys His Arg Lys Leu Val Pro Pro Ser Arg Thr Ser
       130                 135                 140
   Leu Asn Tyr Leu Asp Leu Pro Ala Gly His Ile Gln Ile Phe Thr Thr
   145                 150                 155                 160
   Leu Pro Gln Gly Leu Trp Gln Ile Ser Gly Val Gly Thr Gly Leu Ser
                   165                 170                 175
   Ser Val Leu Val Tyr His Gly Glu Met Pro Gln Val Asn Gln Leu Leu
               180                 185                 190
   Ser Ser Ser Ser Pro Thr Val Pro Ser Leu Pro Glu Ser Pro Asp Arg
           195                 200                 205
   Pro Gly Ser Thr Ser Pro Phe Ala Pro Ser Thr Ala Asp Leu Pro Gly
       210                 215                 220
   Met Pro Glu Pro Ala Leu Thr Ser Arg Val Asn Glu Thr Glu Asp Thr
   225                 230                 235                 240
   Thr Pro Pro Ser Pro Cys Gln Glu Asp Pro Glu Pro Ser Ile Lys Leu
                   245                 250                 255
   Pro Lys Trp Pro Glu Ser Val Glu Arg Phe Gln His Ser Leu Gln Asp
               260                 265                 270
   Lys Tyr Gln Ala Leu Pro Gly Ser Pro Ser Gly Pro Leu Val Ala Met
           275                 280                 285
   Glu Leu Val Arg Ala Arg Leu Glu Arg Gly Ser Asn Lys Ser Gln Glu
       290                 295                 300
   Arg Glu Leu Ala Thr Pro Asp Trp Thr Glu Arg Gln Leu Ala His Gly
   305                 310                 315                 320
   Gly Leu Ala Glu Val Leu Arg Val Val Gly Asp Ser Arg Arg Ser Arg
                   325                 330                 335
   Glu Thr Gln Val Val Ala Val Leu Gly Lys Ala Gly Gln Gly Lys Ser
               340                 345                 350
   His Trp Ala Arg Thr Val Ser His Ile Trp Ala Gln Gly Gln Leu Pro
           355                 360                 365
   Gln Tyr Asp Phe Val Phe Tyr Val Pro Cys Gln Cys Leu Asp Arg Pro
       370                 375                 380
   Gly Asp Thr Tyr His Leu Arg Asp Leu Leu Cys Pro Pro Asn Leu Gln
   385                 390                 395                 400
   Pro Leu Ala Met Asn Asp Glu Val Ile Asp His Ile Val Arg Gln Pro
                   405                 410                 415
   Asp Arg Val Leu Leu Ile Leu Asp Ala Phe Glu Glu Leu Glu Ala Gln
               420                 425                 430
   Asp Ser Leu Leu His Gly Pro Cys Gly Pro Leu Ser Pro Glu Pro Cys
           435                 440                 445
   Ser Leu Arg Gly Leu Leu Ala Gly Leu Phe Gln Arg Lys Leu Leu Arg
       450                 455                 460
   Gly Cys Thr Leu Leu Leu Thr Ala Arg Pro Arg Gly Arg Leu Ala Gln
   465                 470                 475                 480
   Ser Leu Ser Lys Ala Asp Ala Ile Phe Glu Val Pro Ser Phe Ser Thr
                   485                 490                 495
   Lys Gln Ala Gln Ile Tyr Met Arg His Tyr Phe Glu Asn Ser Gly Thr
               500                 505                 510
   Ala Gly Ser His Asp Lys Ala Leu Gly Leu Leu Glu Gly Gln Pro Phe
           515                 520                 525
   Leu Leu Thr His Ser His Ser Pro Val Leu Cys Ser Ala Val Cys Gln
       530                 535                 540
   Leu Ser Lys Ala Leu Leu Asp Gln Gly Ala Gly Ala Gln Leu Pro Cys
   545                 550                 555                 560
   Thr Leu Thr Gly Leu Tyr Val Gly Leu Leu Gly Pro Ala Ala Gln Asn
                   565                 570                 575
   Ser Pro Pro Gly Ala Leu Val Glu Leu Ala Lys Leu Ala Trp Glu Leu
               580                 585                 590
   Gly Arg Arg His Gln Ser Thr Leu Gln Glu Thr Gln Phe Pro Ser Val
           595                 600                 605
   Glu Val Lys Thr Trp Ala Val Ala Gln Gly Leu Met Gln Gln Thr Thr
       610                 615                 620
   Glu Ala Gln Leu Ala Phe Ser Ser Phe Leu Leu Gln Cys Phe Leu Gly
   625                 630                 635                 640
   Ala Val Trp Leu Ala Gln Cys Asn Glu Met Lys Asp Lys Glu Leu Pro
                   645                 650                 655
   Gln Tyr Leu Ala Leu Thr Pro Arg Lys Lys Arg Pro Tyr Asp Asn Trp
               660                 665                 670
   Leu Glu Gly Val Pro Arg Phe Leu Ala Gly Leu Val Phe Gln Pro Arg
           675                 680                 685
   Ala His Cys Leu Gly Ala Leu Val Glu Pro Ala Met Ala Ala Val Ala
       690                 695                 700
   Asp Arg Lys Arg Lys Val Leu Thr Arg Tyr Leu Lys Arg Leu Lys Leu
   705                 710                 715                 720
   Gly Thr Leu Arg Ala Gly Arg Leu Leu Glu Leu Leu His Cys Ala His
                   725                 730                 735
   Glu Thr Gln Glu Pro Gly Ile Trp Lys His Val Ser His Gln Leu Pro
               740                 745                 750
   Gly His Leu Ser Phe Leu Gly Thr Arg Leu Thr Ala Pro Asp Val Tyr
           755                 760                 765
   Val Leu Gly Arg Ala Leu Glu Thr Ala Ser Gln Asp Phe Ser Leu Asp
       770                 775                 780
   Leu Arg Gln Thr Gly Ile Glu Pro Ser Arg Leu Gly Asp Leu Val Gly
   785                 790                 795                 800
   Leu Ser Cys Val Thr Ser Phe Arg Ala Ser Leu Ser Asp Thr Met Ala
                   805                 810                 815
   Leu Trp Glu Ser Leu Gln His Gln Gly Glu Thr Gln Leu Leu Gln Ala
               820                 825                 830
   Ala Glu Glu Lys Phe Thr Ile Glu Pro Phe Lys Ala Lys Ser Pro Lys
           835                 840                 845
   Asp Val Glu Asp Leu Asp Ser Leu Val Gln Thr Gln Arg Leu Arg Asn
       850                 855                 860
   Pro Ser Ala Asp Ala Ala Lys Asp Leu Pro Ala Ile Arg Asp Leu Lys
   865                 870                 875                 880
   Lys Leu Glu Phe Ala Leu Gly Pro Val Leu Gly Pro Gln Ala Phe Pro
                   885                 890                 895
   Thr Leu Ala Lys Ile Leu Pro Ala Phe Ser Ser Leu Gln His Leu Asp
               900                 905                 910
   Leu Asp Ser Leu Ser Glu Asn Lys Ile Gly Asp Lys Gly Val Ser Lys
           915                 920                 925
   Leu Ser Ala Thr Phe Pro Gln Leu Lys Ala Leu Glu Thr Leu Asn Leu
   930 935 940
   Ser Gln Asn Ser Ile Thr Asp Val Gly Ala Cys Lys Leu Ala Glu Ala
   945 950 955 960
   Leu Pro Ala Leu Ala Lys Ser Leu Leu Arg Leu Ser Leu Tyr Asn Asn
   965 970 975
   Cys Ile Cys Asp Glu Gly Ala Lys Ser Leu Ala Arg Val Leu Pro Asp
   980 985 990
   Met Val Ser Leu Arg Val Met Asp Val Gln Phe Asn Lys Phe Thr Ala
   995 1000 1005
   Val Gly Ala Gln Gln Leu Thr Ser Ser Leu Gln Lys Cys Pro Gln Val
   1010 1015 1020
   Glu Thr Leu Ala Met Trp Thr Pro Thr Ile Pro Phe Gly Val Gln Glu
   1025 1030 1035 1040
   His Leu Gln Gln Leu Asp Ala Arg Ile Ser Leu Arg
   1045 1050

<210> 35
<211> 119
<212> PRT
<213> Artificial Sequence

<220>
<223> [CDS]:1..360 from SEQ ID NO 28

<400> 35
Met Ser Arg Ser Val Ala Leu Ala Val Leu Ala Leu Leu Ser Leu Ser
1               5                   10                  15
Gly Leu Glu Ala Ile Gln Arg Thr Pro Lys Ile Gln Val Tyr Ser Arg
            20                  25                  30
His Pro Ala Glu Asn Gly Lys Ser Asn Phe Leu Asn Cys Tyr Val Ser
        35                  40                  45
Gly Phe His Pro Ser Asp Ile Glu Val Asp Leu Leu Lys Asn Gly Glu
    50                  55                  60
Arg Ile Glu Lys Val Glu His Ser Asp Leu Ser Phe Ser Lys Asp Trp
65                  70                  75                  80
Ser Phe Tyr Leu Leu Tyr Tyr Thr Glu Phe Thr Pro Thr Glu Lys Asp
                85                  90                  95
Glu Tyr Ala Cys Arg Val Asn His Val Thr Leu Ser Gln Pro Lys Ile
            100                 105                 110
Val Lys Trp Asp Arg Asp Met
        115

<210> 36
<211> 1130
<212> PRT
<213> Artificial Sequence

<220>
<223> [CDS]:1..3393 from SEQ ID NO 29

<400> 36
Met Arg Cys Leu Ala Pro Arg Pro Ala Gly Ser Tyr Leu Ser Glu Pro
1               5                   10                  15
Gln Gly Ser Ser Gln Cys Ala Thr Met Glu Leu Gly Pro Leu Glu Gly
            20                  25                  30
Gly Tyr Leu Glu Leu Leu Asn Ser Asp Ala Asp Pro Leu Cys Leu Tyr
        35                  40                  45
His Phe Tyr Asp Gln Met Asp Leu Ala Gly Glu Glu Glu Ile Glu Leu
    50                  55                  60
Tyr Ser Glu Pro Asp Thr Asp Thr Ile Asn Cys Asp Gln Phe Ser Arg
65                  70                  75                  80

Leu Leu Cys Asp Met Glu Gly Asp Glu Glu Thr Arg Glu Ala Tyr Ala
                85                  90                  95
Asn Ile Ala Glu Leu Asp Gln Tyr Val Phe Gln Asp Ser Gln Leu Glu
            100                 105                 110
Gly Leu Ser Lys Asp Ile Phe Lys His Ile Gly Pro Asp Glu Val Ile
        115                 120                 125
Gly Glu Ser Met Glu Met Pro Ala Glu Val Gly Gln Lys Ser Gln Lys
    130                 135                 140
Arg Pro Phe Pro Glu Glu Leu Pro Ala Asp Leu Lys His Trp Lys Pro
145                 150                 155                 160
Ala Glu Pro Pro Thr Val Val Thr Gly Ser Leu Leu Val Gly Pro Val
                165                 170                 175
Ser Asp Cys Ser Thr Leu Pro Cys Leu Pro Leu Pro Ala Leu Phe Asn
            180                 185                 190
Gln Glu Pro Ala Ser Gly Gln Met Arg Leu Glu Lys Thr Asp Gln Ile
        195                 200                 205
Pro Met Pro Phe Ser Ser Ser Ser Leu Ser Cys Leu Asn Leu Pro Glu
    210                 215                 220
Gly Pro Ile Gln Phe Val Pro Thr Ile Ser Thr Leu Pro His Gly Leu
225                 230                 235                 240
Trp Gln Ile Ser Glu Ala Gly Thr Gly Val Ser Ser Ile Phe Ile Tyr
                245                 250                 255
His Gly Glu Val Pro Gln Ala Ser Gln Val Pro Pro Pro Ser Gly Phe
            260                 265                 270
Thr Val His Gly Leu Pro Thr Ser Pro Asp Arg Pro Gly Ser Thr Ser
        275                 280                 285
Pro Phe Ala Pro Ser Ala Thr Asp Leu Pro Ser Met Pro Glu Pro Ala
    290                 295                 300
Leu Thr Ser Arg Ala Asn Met Thr Glu His Lys Thr Ser Pro Thr Gln
305                 310                 315                 320
Cys Pro Ala Ala Gly Glu Val Ser Asn Lys Leu Pro Lys Trp Pro Glu
                325                 330                 335
Pro Val Glu Gln Phe Tyr Arg Ser Leu Gln Asp Thr Tyr Gly Ala Glu
            340                 345                 350
Pro Ala Gly Pro Asp Gly Ile Leu Val Glu Val Asp Leu Val Gln Ala
        355                 360                 365
Arg Leu Glu Arg Ser Ser Ser Lys Ser Leu Glu Arg Glu Leu Ala Thr
    370                 375                 380
Pro Asp Trp Ala Glu Arg Gln Leu Ala Gln Gly Gly Leu Ala Glu Val
385                 390                 395                 400
Leu Leu Ala Ala Lys Glu His Arg Arg Pro Arg Glu Thr Arg Val Ile
                405                 410                 415
Ala Val Leu Gly Lys Ala Gly Gln Gly Lys Ser Tyr Trp Ala Gly Ala
            420                 425                 430
Val Ser Arg Ala Trp Ala Cys Gly Arg Leu Pro Gln Tyr Asp Phe Val
        435                 440                 445
Phe Ser Val Pro Cys His Cys Leu Asn Arg Pro Gly Asp Ala Tyr Gly
    450                 455                 460
Leu Gln Asp Leu Leu Phe Ser Leu Gly Pro Gln Pro Leu Val Ala Ala
465                 470                 475                 480
Asp Glu Val Phe Ser His Ile Leu Lys Arg Pro Asp Arg Val Leu Leu
                485                 490                 495
Ile Leu Asp Gly Phe Glu Glu Leu Glu Ala Gln Asp Gly Phe Leu His
            500                 505                 510
Ser Thr Cys Gly Pro Ala Pro Ala Glu Pro Cys Ser Leu Arg Gly Leu
        515                 520                 525
Leu Ala Gly Leu Phe Gln Lys Lys Leu Leu Arg Gly Cys Thr Leu Leu
    530                 535                 540
Leu Thr Ala Arg Pro Arg Gly Arg Leu Val Gln Ser Leu Ser Lys Ala
545                 550                 555                 560
Asp Ala Leu Phe Glu Leu Ser Gly Phe Ser Met Glu Gln Ala Gln Ala
                565                 570                 575
Tyr Val Met Arg Tyr Phe Glu Ser Ser Gly Met Thr Glu His Gln Asp

            580                 585                 590
Arg Ala Leu Thr Leu Leu Arg Asp Arg Pro Leu Leu Leu Ser His Ser
        595                 600                 605
His Ser Pro Thr Leu Cys Arg Ala Val Cys Gln Leu Ser Glu Ala Leu
    610                 615                 620
Leu Glu Leu Gly Glu Asp Ala Lys Leu Pro Ser Thr Leu Thr Gly Leu
625                 630                 635                 640
Tyr Val Gly Leu Leu Gly Arg Ala Ala Leu Asp Ser Pro Pro Gly Ala
                645                 650                 655
Leu Ala Glu Leu Ala Lys Leu Ala Trp Glu Leu Gly Arg Arg His Gln
            660                 665                 670
Ser Thr Leu Gln Glu Asp Gln Phe Pro Ser Ala Asp Val Arg Thr Trp
        675                 680                 685
Ala Met Ala Lys Gly Leu Val Gln His Pro Pro Arg Ala Ala Glu Ser
    690                 695                 700
Glu Leu Ala Phe Pro Ser Phe Leu Leu Gln Cys Phe Leu Gly Ala Leu
705                 710                 715                 720
Trp Leu Ala Leu Ser Gly Glu Ile Lys Asp Lys Glu Leu Pro Gln Tyr
                725                 730                 735
Leu Ala Leu Thr Pro Arg Lys Lys Arg Pro Tyr Asp Asn Trp Leu Glu
            740                 745                 750
Gly Val Pro Arg Phe Leu Ala Gly Leu Ile Phe Gln Pro Pro Ala Arg
        755                 760                 765
Cys Leu Gly Ala Leu Leu Gly Pro Ser Ala Ala Ala Ser Val Asp Arg
    770                 775                 780
Lys Gln Lys Val Leu Ala Arg Tyr Leu Lys Arg Leu Gln Pro Gly Thr
785                 790                 795                 800
Leu Arg Ala Arg Gln Leu Leu Glu Leu Leu His Cys Ala His Glu Ala
                805                 810                 815
Glu Glu Ala Gly Ile Trp Gln His Val Val Gln Glu Leu Pro Gly Arg
            820                 825                 830
Leu Ser Phe Leu Gly Thr Arg Leu Thr Pro Pro Asp Ala His Val Leu
        835                 840                 845
Gly Lys Ala Leu Glu Ala Ala Gly Gln Asp Phe Ser Leu Asp Leu Arg
    850                 855                 860
Ser Thr Gly Ile Cys Pro Ser Gly Leu Gly Ser Leu Val Gly Leu Ser
865                 870                 875                 880
Cys Val Thr Arg Phe Arg Ala Ala Leu Ser Asp Thr Val Ala Leu Trp
                885                 890                 895
Glu Ser Leu Gln Gln His Gly Glu Thr Lys Leu Leu Gln Ala Ala Glu
            900                 905                 910
Glu Lys Phe Thr Ile Glu Pro Phe Lys Ala Lys Ser Leu Lys Asp Val
        915                 920                 925
Glu Asp Leu Gly Lys Leu Val Gln Thr Gln Arg Thr Arg Ser Ser Ser
    930                 935                 940
Glu Asp Thr Ala Gly Glu Leu Pro Ala Val Arg Asp Leu Lys Lys Leu
945                 950                 955                 960
Glu Phe Ala Leu Gly Pro Val Ser Gly Pro Gln Ala Phe Pro Lys Leu
                965                 970                 975
Val Arg Ile Leu Thr Ala Phe Ser Ser Leu Gln His Leu Asp Leu Asp
            980                 985                 990
Ala Leu Ser Glu Asn Lys Ile Gly Asp Glu Gly Val Ser Gln Leu Ser
        995                 1000                1005
Ala Thr Phe Pro Gln Leu Lys Ser Leu Glu Thr Leu Asn Leu Ser Gln
    1010                1015                1020
Asn Asn Ile Thr Asp Leu Gly Ala Tyr Lys Leu Ala Glu Ala Leu Pro
1025                1030                1035                1040
Ser Leu Ala Ala Ser Leu Leu Arg Leu Ser Leu Tyr Asn Asn Cys Ile
                1045                1050                1055
Cys Asp Val Gly Ala Glu Ser Leu Ala Arg Val Leu Pro Asp Met Val
            1060                1065                1070
Ser Leu Arg Val Met Asp Val Gln Tyr Asn Lys Phe Thr Ala Ala Gly
        1075                1080                1085

Ala Gln Gln Leu Ala Ala Ser Leu Arg Arg Cys Pro His Val Glu Thr
    1090                1095                1100
Leu Ala Met Trp Thr Pro Thr Ile Pro Phe Ser Val Gln Glu His Leu
1105                1110                1115                1120
Gln Gln Gln Asp Ser Arg Ile Ser Leu Arg
                1125                1130

<210> 37
<211> 118
<212> PRT
<213> Artificial Sequence

<220>
<223> [CDS]:1..357 from SEQ ID NO 30

<400> 37
Met Ala Pro Leu Val Ala Leu Val Leu Leu Gly Leu Leu Ser Leu Ser
1               5                   10                  15
Gly Leu Asp Ala Val Ala Arg Pro Pro Lys Val Gln Val Tyr Ser Arg
            20                  25                  30
His Pro Ala Glu Asn Gly Lys Pro Asn Tyr Leu Asn Cys Tyr Val Ser
        35                  40                  45
Gly Phe His Pro Pro Gln Ile Glu Ile Asp Leu Leu Lys Asn Gly Glu
    50                  55                  60
Lys Met Asn Ala Glu Gln Ser Asp Leu Ser Phe Ser Lys Asp Trp Ser
65                  70                  75                  80
Phe Tyr Leu Leu Val His Thr Glu Phe Thr Pro Asn Ala Val Asp Gln
                85                  90                  95
Tyr Ser Cys Arg Val Lys His Val Thr Leu Asp Lys Pro Lys Ile Val
            100                 105                 110
Lys Trp Asp Arg Asp His
        115

<210> 38
<211> 898
<212> PRT
<213> Artificial Sequence

<220>
<223> [CDS]:1..2697 from SEQ ID NO 31

<400> 38
Met Glu Leu Gly Pro Leu Glu Gly Gly Tyr Leu Glu Leu Leu Asn Ser
1               5                   10                  15
Ser Ala Asp Pro Leu Gln Leu Tyr His Leu Tyr Asp Arg Met Asp Leu
            20                  25                  30
Ala Gly Glu Glu Glu Ile Glu Leu Cys Ser Glu Pro Asp Thr Asp Thr
        35                  40                  45
Ile Asn Cys Glu Gln Phe Ser Arg Leu Leu Cys Asp Met Glu Ala Asp
    50                  55                  60
Glu Glu Thr Arg Glu Thr Tyr Ala Ser Ile Ala Glu Leu Asp Gln Tyr
65                  70                  75                  80
Val Phe Gln Asp Ser Gln Leu Glu Gly Leu Gly Lys Asp Ile Phe Ile
                85                  90                  95
Glu His Ile Gly Leu Glu Glu Met Ile Ser Glu Ser Val Glu Val Leu
            100                 105                 110
Glu Asp Ser Gly Arg Lys Ser Gln Lys Arg Ser Phe Pro Glu Glu Leu
        115                 120                 125
Pro Ala Asp Leu Lys His Arg Lys Leu Ala Glu Pro Leu Ala Met Pro
    130                 135                 140
Met Val Thr Gly Thr Phe Leu Val Gly Pro Val Ser Asp Ser Ser Ala

145                 150                 155                 160
Arg Pro Cys Pro Ser Pro Pro Ala Leu Phe Asn Lys Glu Ser Thr Pro
                165                 170                 175
Ser Gln Ala Gln Leu Glu Asp Ala Val Pro Met Pro Ala Pro Pro Ser
            180                 185                 190
Gly Ser Leu Leu Ser Cys Leu Ser Val Pro Ala Gly Pro Ile Gln Ile
        195                 200                 205
Ile Pro Thr Leu Ser Thr Leu Pro Gln Gly Leu Trp His Ile Ser Gly
    210                 215                 220
Ala Gly Thr Gly Val Ser Ser Ile Leu Ile Tyr Gln Gly Glu Arg Gly
225                 230                 235                 240
Lys Pro Gly Ser Pro Pro Pro Leu Pro Val Thr Pro Val Leu Pro Leu
                245                 250                 255
Ala Ser Ser Pro Pro Pro Leu Pro Pro Leu Thr Cys Ala Glu Thr Val
            260                 265                 270
Glu Gln Phe His His Ser Leu Arg Asp Arg Tyr Gln Ala Lys Pro Ala
        275                 280                 285
Gly Pro Glu Gly Ile Leu Val Glu Val Asp Leu Val Arg Val Arg Leu
    290                 295                 300
Glu Arg Ser Ser Ser Lys Ser Gln Glu Arg Glu Leu Ala Ser Leu Asp
305                 310                 315                 320
Trp Ala Glu Arg Gln Pro Ala Arg Gly Gly Leu Ala Glu Val Leu Leu
                325                 330                 335
Ala Ala Ser Asp Arg Gln Gly Pro Arg Glu Thr Gln Val Ile Ala Val
            340                 345                 350
Leu Gly Lys Ala Gly Gln Gly Lys Ser His Trp Ala Gln Ala Val Ser
        355                 360                 365
Trp Ala Trp Ala Asp Gly Gln Leu Pro Gln Tyr Asp Phe Val Phe Cys
    370                 375                 380
Ile Pro Cys His Cys Leu Asp Arg Pro Gly Asn Thr Tyr Arg Leu Gln
385                 390                 395                 400
Asp Leu Leu Phe Ser Leu Gly Pro Gln Pro Leu Pro Met Asp Asp Glu
                405                 410                 415
Val Phe Ser Tyr Ile Leu Arg Arg Pro Asp Arg Val Leu Leu Ile Leu
            420                 425                 430
Asp Ala Phe Glu Glu Arg Glu Ala Gln Asp Gly Phe Val His Ser Ala
        435                 440                 445
Gly Gly Pro Leu Ser Ser Glu Pro Arg Ser Leu Arg Gly Leu Leu Ala
    450                 455                 460
Gly Leu Leu Gln Arg Lys Leu Leu Arg Gly Cys Thr Leu Leu Leu Thr
465                 470                 475                 480
Ala Arg Pro Arg Gly Arg Leu Ala Gln Ser Leu Ser Lys Ala Asp Ala
                485                 490                 495
Leu Phe Glu Val Ala Gly Phe Ser Ala Gln Gln Ala Lys Thr Tyr Met
            500                 505                 510
Leu Arg Tyr Phe Glu Cys Arg Gly Ala Arg Glu Arg Gln Lys Arg Ala
        515                 520                 525
Leu Glu Leu Leu Gln Ala Gln Pro Phe Leu Leu Ser His Ser His Ser
    530                 535                 540
Pro Ser Val Cys Arg Ala Val Cys Arg Leu Ser Glu Thr Leu Leu Glu
545                 550                 555                 560
Leu Gly Glu Glu Ala Glu Leu Pro Ser Thr Leu Thr Gly Leu Tyr Val
                565                 570                 575
Gly Leu Leu Gly Pro Ala Ala Arg Glu Ser Pro Pro Gly Ala Leu Val
            580                 585                 590
Gly Leu Ala Arg Leu Ala Trp Glu Leu Gly Arg Arg His His Ser Ser
        595                 600                 605
Leu Gln Glu Gly Gln Phe Pro Ser Ala Glu Ala Arg Ala Trp Ala Val
    610                 615                 620
Ala Gln Gly Leu Val Gln Arg Ala Pro Gly Ala Pro Gly Ala Pro Glu
625                 630                 635                 640
Leu Ala Phe Ser Ser Phe Leu Leu Gln Cys Phe Leu Gly Ala Val Trp
                645                 650                 655

Leu Ala Leu Ser Ser Glu Ile Lys Asp Lys Glu Leu Pro Gln Tyr Leu
            660                 665                 670
Ala Leu Thr Pro Arg Lys Lys Arg Pro Tyr Asp Asn Trp Leu Glu Ala
        675                 680                 685
Val Pro Arg Phe Leu Val Gly Leu Val Phe Gln Pro Arg Ala Arg Cys
    690                 695                 700
Leu Gly Ala Leu Ala Gly Leu Val Ala Ala Thr Leu Ala Asp Arg Lys
705                 710                 715                 720
Gln Lys Val Leu Asn Arg Tyr Leu Lys Arg Leu Gln Pro Gly Thr Leu
                725                 730                 735
Gln Ala Gly Arg Leu Leu Glu Leu Leu His Cys Thr His Glu Ala Leu
            740                 745                 750
Asp Ser Gly Leu Trp Gln His Val Leu Gln Gly Leu Pro Thr Gln Leu
        755                 760                 765
Ser Phe Leu Gly Thr Arg Leu Thr Pro Pro Asp Thr His Val Leu Gly
    770                 775                 780
Ser Ala Leu Val Ala Ala Gly Arg Asp Phe Ser Leu Asp Leu Arg Ser
785                 790                 795                 800
Thr Gly Ile Asp Pro Ser Gly Leu Gly Ser Leu Val Gly Leu Ser Cys
                805                 810                 815
Val Thr His Phe Arg Ala Ala Leu Ser Asp Thr Val Gly Leu Trp Glu
            820                 825                 830
Ser Leu Gln Gln Arg Gly Glu Thr Lys Leu Leu Gln Ala Leu Glu Glu
        835                 840                 845
Lys Phe Thr Ile Glu Pro Phe Lys Ala Lys Ser Met Lys Asp Val Glu
    850                 855                 860
Asp Leu Gly Asn Leu Val Gln Ile Gln Arg Thr Arg Ser Ser Ser Glu
865                 870                 875                 880
Asp Met Ala Gly Glu Leu Pro Ala Val Arg Asp Leu Lys Lys Leu Glu
                885                 890                 895
Phe Ala

## Description

The present invention relates to a tissue for use as a transplant, which tissue is allogeneic or xenogeneic and respectively the tissue may express an MHC I molecule that is immunologically incompatible to the transplant recipient and/or may express an MHC II molecule immunologically incompatible to the transplant recipient. The tissue may alternatively express identical MHC I and MHC II molecules, as even identical MHC molecules in twins can present different peptides, which in transplantation result in minor histocompatibility antigens. The tissue suitable for use as a transplant and the method for its production comprise a genetic alteration of the tissue that provides for immunologic compatibility of the tissue with a transplant recipient. Further, the invention relates to nucleic acid constructs, e.g. viral vectors, suitable for introducing the genetic alterations into allogeneic or xenogeneic tissue in order to make them immunologically compatible to a transplant recipient.

The tissue comprising the genetic alteration according to the invention, and respectively tissue produced according to the invention, in spite of being allogeneic or in spite of the expression of allogeneic or xenogeneic MHC I and MHC II molecules has the advantage of being immunologically tolerated by the transplant recipient, and that therefore no or a reduced general immune suppression of the transplant recipient is required in order to avoid cellular and/or humoral transplant rejection (host-versus-graft (HvG) disease). A further advantage of the tissue according to the invention is that the immune system of the transplant recipient does not need to be modulated in the same extent as usually required, e.g. in an allotransplantation setting, e.g. by using powerful immunosuppressants. Accordingly, the genetically altered tissue according to the invention is suitable for use as a transplant in a recipient without temporary or permanent standard immune suppression, e.g. without or with reduced immunosuppressant for temporary or for permanent use in the treatment of adverse immune reactions, e.g. in the treatment of adverse immune reactions against the transplant, especially HvG disease. Preferably, the genetically altered tissue according to the invention is for use as a transplant with reduced or without general or specific immune suppression, e.g. excluding pharmaceutical compounds, e.g. immunosuppressants, for use in suppressing immunological reactions, e.g. for suppressing HvG disease, wherein the use in suppressing immunological reactions is preferably temporary, more preferably permanent.

### State of the art

It is known that graft rejection by the recipient of the grafted transplant is a complex multifactorial process that results in the destruction of the transplanted tissue by the immune response of the recipient. This immune response is led by T-cells that directly or indirectly recognise MHC molecules of the transplant as allogeneic or xenogeneic. Also, B-cells are activated which leads to the production of antibodies which usually are directed against the immunologically incompatible MHC molecules of the transplant.

The tissue that is transplanted can easily be immunologically incompatible in respect of the recipient due to differences in its MHC class I or MHC class II antigens and/or in its allogeneic or xenogeneic peptides presented in even identical MHC molecules. A standard practice for reducing the immune reaction of the transplant recipient against the transplant (HvG disease) is a general and unspecific immune suppression of the transplant recipient, which in turn leads to severe side effects and to frequent occurrence of e.g. infections and increased risk of tumours.

EP 1546174 B1 describes generally that nucleic acid constructs encoding short hairpin RNA upon expression in a cell specifically suppress the expression of a hybridizing target nucleic acid.

WO 2018/215571 A1 describes a method for genetically modifying a vascularized tissue by an ex vivo method in order to genetically modify the vascularized tissue, which may be an organ, for use as a transplant.

### Object of the invention

It is an object of the invention to provide a tissue that expresses immunologically incompatible or compatible MHC I and/or immunologically incompatible or compatible MHC II molecules for use as a transplant in a future transplant recipient, which tissue is tolerated by the recipient. A further object is to provide an ex vivo method for manipulating an allogeneic or xenogeneic tissue to produce a tissue that is immunologically tolerated by a future transplant recipient and is therefore suitable for use as a transplant. A further object is to provide compounds, e.g. nucleic acid constructs, preferably packaged in viral particles, which are suitable for the manipulation of the allogeneic or xenogeneic tissue in order to make the tissue immunologically tolerable.

### Description of the invention

The invention achieves the objects by the features of the claims, and specifically provides nucleic acid constructs which are suitable as compounds for the treatment of HvG disease, e.g. for making allogeneic or xenogeneic tissue immunologically tolerable, especially tolerable for a recipient of the allogeneic or xenogeneic tissue. Further, the invention provides allogeneic or xenogeneic tissue, e.g. tissue expressing an MHC I molecule and/or an MHC II molecule, which is genetically manipulated to make the tissue tolerable to the immune system of a recipient, and therefore the invention provides the genetically manipulated tissue for use as a transplant. Further, the invention provides an in vitro process for producing the tissue for use as a transplant.

Herein, tissue expressing an MHC I molecule and/or an MHC II molecule having immunologically detectable differences from the MHC molecules or the peptides presented therein of a recipient, and/or which MHC I molecule and/or MHC II molecule or the peptides presented therein would be recognized as non-self by a recipient and would elicit an immune reaction against the MHC I molecule and/or MHC II molecule, is referred to as allogeneic or xenogeneic.

In transplant recipients, it was found that the downregulation of allogeneic MHC I molecule expression abrogates the generation of cytotoxic T-cells when the expression of the MHC I molecule is suppressed by at least 60% or by at least 70% or by at least 80% in comparison to wild-type cells, leaving a residual expression of at most 40%, respectively of at most 30% or of at most 20%. However, it was also found that the downregulation of the expression of allogeneic MHC I induces the generation or activation of cytotoxic NK-cells, e.g. when the suppression of the allogeneic MHC I molecule is by at least 80%, e.g. by 80 to 90%, compared to wild-type cells, leaving a residual expression of 20 to 10%.

The downregulation of expression of allogeneic MHC II molecules by at least 70%, e.g. by 70% up to at least 80% or more, leaving a residual expression of at most 30%, e.g. 30 to 20% or less, in comparison to expression of MHC II in wild-type cells, in transplant recipients was found to be sufficient to support the expansion of regulatory T-cells (Treg cells) of the recipients, e.g. in contrast to HLA-DR knockout cells which are not able to induce the expansion Treg cells.

In respect of antibody-mediated cytotoxicity, it was found in transplant recipients that a downregulation of the expression of allogeneic MHC I and of the allogeneic MHC II molecules by at least 70% or by at least or by at least 90%, leaving a residual expression of at most 30% or 20% or 10%, compared to wild-type cells, abrogates antibody-mediated cytotoxicity, even though antibodies directed against the MHC were generated.

It was found further in transplant recipients that the downregulation of the expression of allogeneic MHC I and of allogeneic MHC II by about 100%, i.e. to about 0% residual expression, compared to wild-type cells, abrogates formation of antibodies directed against the MHC I and of antibodies directed against the MHC II.

In order to provide for immunologic tolerance of a tissue for use as a transplant, which tissue expresses allogeneic or xenogeneic MHC I and/or allogeneic or xenogeneic MHC II molecules, which tolerance includes avoidance and prevention of an adverse immune reaction by a transplant recipient, the tissue of the invention is genetically manipulated, e.g. to quantitatively balance the level of expression of the allogeneic or xenogeneic MHC I and/or allogeneic or xenogeneic MHC II molecules, and to prevent a complement reaction.

The invention provides a tissue for use as a transplant, which tissue expresses identical or allogeneic or xenogeneic MHC I and/or identical or allogeneic or xenogeneic MHC II molecules,
wherein the expression of the identical or allogeneic or xenogeneic MHC I is downregulated by at least 50% to up to 90%, leaving a residual expression of at maximum 50% to 10%, wherein preferably the expression of the identical or allogeneic or xenogeneic MHC I is downregulated by at least 60%, more preferably by at least 70%, more preferably by 80% up to 90% or up to 85%, leaving a residual expression of the allogeneic or xenogeneic MHC I of at maximum 40%, at maximum 30%, more preferably at maximum 20% to 10% or 20% to 15%,
and wherein the expression of the identical or allogeneic or xenogeneic MHC II is downregulated by at least 50%, e.g. by at least 60%, e.g. downregulated by up to 90%, preferably downregulated by at least 70%, more preferably downregulated by at least 80%, e.g. downregulated by up to 90% or by up to 85%, leaving a residual expression of the identical or allogeneic or xenogeneic MHC II of at maximum 50%, e.g. of at maximum 40%, e.g. of at least 10%, preferably of at maximum 30%, more preferably of at maximum 20%, e.g. a residual expression of at least 10% or at least 15%,
wherein the downregulation of the identical or allogeneic or xenogeneic MHC I and/or of the identical or allogeneic or xenogeneic MHC II is in relation to its expression in wild-type tissue,
and additionally expressing at least one complement inhibitory molecule on the tissue, which complement inhibitory molecule is at least one of CD46, CD55 and CD59, preferably two of these, more preferably expression of CD46 and of CD55 and of CD59. Generally preferred, in the tissue for use as a transplant, which tissue expresses MHC I and MHC II molecules, the expression of the MHC I is downregulated by at least 50% to up to 90% and the expression of MHC I is downregulated by at least 60%. Herein, the term allogeneic MHC I and MHC II may comprise identical MHC I and identical MHC II, respectively.

Herein, the upper and lower percentages can be combined freely to define ranges of downregulation, respectively of residual expression.

Herein, expression in wild-type is the level of expression in the tissue prior to genetic manipulation, or the level of expression by the transplant recipient's own tissue, e.g. autologous tissue of the transplant recipient. Alternatively, e.g. using expression cassettes encoding inhibitory RNA for downregulation of MHC transcripts, downregulation can be determined as reduction of transcript levels, preferably normalized to transcript levels of a household gene. Transcript levels can quantitatively be determined by RT-PCR, allowing the determination of residual transcript levels that remain after hybridization with inhibitory RNA, and residual transcript levels are a measure for expression levels.

Upon transplantation into a recipient, the tissue according to the invention does not abrogate the activation and expansion of Treg-cells and prevents or avoids the generation or activation of cytotoxic T-cells, of cytotoxic NK-cells and delays or prevents or avoids the formation of antibodies originating from B-cells expressing antibody directed against the allogeneic or xenogeneic MHC I and/or directed against the allogeneic or xenogeneic MHC II.

Accordingly, the downregulation of the expression of the allogeneic or xenogeneic MHC I and/or of the allogeneic or xenogeneic MHC II in the tissue provides for tolerance by the immune system of the recipient of the tissue or mitigates the immune reaction against the tissue.

As residual expression of MHC molecules has been found to be essential to prevent NK cell-mediated responses and support Treg cell activity, the formation of antibodies may not completely be prevented. The expression of complement inhibitors was found to inhibit the cytotoxic effect of antibodies directed against the allogeneic or xenogeneic MHC I or against the allogeneic or xenogeneic MHC II molecules efficiently, e.g. co-expression of complement inhibitors CD55 and CD59, or co-expression of CD46 and CD55, or co-expression of CD46 an CD59, preferably of CD46 and of CD55 and of CD59 in the tissue, only or particularly when the expression of the allogeneic or xenogeneic MHC I and of the allogeneic or xenogeneic MHC II is downregulated.

Expression levels of MHC II and of MHC I can be determined e.g. by FACS using antibodies directed against MHC II, respectively against MHC I, using isotype controls as reference to determine the background fluorescence. Transcripts levels of MHC II and of MHC I can be determined e.g. by Real-Time PCR using primers and probes directed against MHC II, respectively against MHC I, preferably using expression of a household gene as an internal standard and normalizing expression of MHC II, respectively of MHC I, to the expression of the household gene. A suitable household gene is e.g. beta-actin, the expression of which can be determined for normalizing expression of MHC II and of MHC I, in wild-type tissue and in tissue genetically manipulated with nucleic acid constructs that downregulate expression of MHC II and of MHC I, respectively.

The MHC I and the MHC II of the tissue is allogenic or xenogeneic in respect of the transplant recipient. For example, the MHC which is xenogeneic can be a swine leukocyte antigen (SLA) for a non-swine recipient, e.g. for a human recipient, and an allogeneic MHC can be a human leukocyte antigen (HLA) which differs from the HLA type of the human recipient and/or differs by presenting allogeneic peptides from the recipient even if the donor and recipient have the same HLA type.

It was found that the balanced downregulation of the allogeneic or xenogeneic MHC I expression by at least 60% of the expression level compared to wild-type expression, and by at maximum 90%, leaving a residual expression of at least 40% to 10%, compared to expression of the wild-type, in combination with a downregulation of the expression of the allogeneic or xenogeneic MHC II by at least 50% up to 80%, leaving a residual MHC II expression of at least 50% to 20%, compared to expression of the wild-type, significantly reduces the interaction of cytotoxic T-cells, e.g. via their T-cell receptor (TCR), but enables the interaction of the Treg cells via their TCR with MHC II, and enables the interaction of NK-cells with MHC I to a sufficient extent to prevent cytotoxicity by NK-cells. The residual expression of the allogeneic or xenogeneic MHC I and of the allogeneic or xenogeneic MHC II results in generation of antibody producing cells, which antibodies are directed against the MHC I and/or against the MHC II. The expression of at least one molecule, preferably of two or of all three molecules, selected from CD46, CD55 and CD59, on the tissue has been found to prevent detrimental effects of these antibodies, e.g. to provide protection against complement activity against the tissue.

In addition, the tissue can be genetically manipulated to downregulate expression of gene products other than MHC I and MHC II, which gene products are foreign to the transplant recipient, e.g. downregulated by at least 80%, preferably by at least 90%, more preferably downregulated by 100%.

For downregulation of the expression of the allogeneic or xenogeneic MHC I and of the allogeneic or xenogeneic MHC II, as well as for expression of at least one of the complement inhibitory molecules the tissue can be genetically manipulated by integration of nucleic acid constructs which encode inhibitory RNA sequences, e.g. siRNA, preferably short hairpin RNA (shRNA), which downregulate the expression of MHC class I and MHC class II genes, e.g. by being specific for the allogeneic or xenogeneic MHC I gene transcripts, respectively for the allogeneic and/or xenogeneic MHC II gene transcripts. Accordingly, in the tissue the expression of the allogeneic or xenogeneic MHC I and/or the expression of the allogeneic or xenogeneic MHC II is downregulated by the tissue being genetically manipulated to contain at least one nucleic acid construct comprising at least one expression cassette encoding inhibitory RNA interfering with at least one transcript encoding the allogeneic or xenogeneic MHC I molecule and/or encoding the allogeneic or xenogeneic MHC II molecule.

For downregulation of MHC I expression, the inhibitory RNA, e.g. siRNA, preferably shRNA is preferably specific for the transcript of the β2-microglobulin gene (β2m), and/or specific for the transcript of at least one, or two, preferably for transcripts of all three heavy chains of e.g. HLA-A, HLA-B and HLA-C molecules or SLA-1, SLA-2 and SLA-3 molecules.

For downregulation of MHC II expression, the inhibitory RNA sequences preferably are specific for transcripts of at least one of the alpha chain and/or beta chain of e.g. HLA-DR, HLA-DQ and HLA-DP or SLA-DR and SLA-DQ, and/or by encoding inhibitory RNA sequences which are specific for the transcripts of the class II transactivator gene (CIITA), e.g. shRNA.

Generally, inhibitory RNA sequences can comprise one or more RNA sequences which are specific for one or more different transcripts.

In order to downregulate as described herein, the inhibitory RNA sequences, e.g. shRNA sequences, can have sequences which only suboptimally hybridize, e.g. by being less specific for the transcript, by designing the inhibitory RNA for hybridizing to less effective regions of the transcripts than SEQ ID NO: 1, which optimally hybridizes to the mRNA of shβ2m and may inhibit higher than desired in the invention, or SEQ ID NO: 3, which optimally hybridizes to the mRNA of CIITA, e.g. SEQ ID NO: 2 (HS_shβ2m-2), which according to the invention less efficiently hybridizes to the mRNA of β2m, leading to a HLA class I suppression of only up to 60%, or SEQ ID NO: 4 (HS_shCIITA-2), which according to the invention less efficiently hybridizes to the mRNA of CIITA, leading to a HLA class II expression of only up to 60%, by controlling transcription of the inhibitory RNA sequences by using a promoter having an activity to express the inhibitory RNA only at the activity required for the downregulation, e.g. by using the U6 sequence, SEQ ID NO: 15, causing a 10%-20% weaker expression of the shRNAs than e.g. a H1 promoter, having SEQ ID NO: 14, or by the inhibitory RNA sequences being shorter than the optimal inhibitory RNA, e.g. SEQ ID NO: 5 for human CIITA (HS_shCIITA-3), or by the inhibitory RNA having one or more mismatches to the mRNA encoding MHC I or MHC II, e.g. one or more mismatches in the hybridizing section, e.g. according to the invention SEQ ID NO: 7 (SS_shβ2m-2) for the Sus scrofa β2m transcript. The efficacy of hybridization of inhibitory RNA to a transcript of the allogeneic or xenogeneic MHC I and/or MHC II molecules can be determined, e.g. by calculating or experimentally determining the stability of the hybrid, resp. determining the efficacy of RNA interference, e.g. by RT-PCR or flow cytometry. The human mRNA sequence encoding β2m is SEQ ID NO: 28, the human mRNA sequence encoding CIITA is SEQ ID NO: 29.

The design of a less effective inhibitory RNA, e.g. a shRNA, can be performed by in vitro experiments and by the support of RNAi designer software tools, e.g. by the publicly accessible tool BLOCK-iT™ RNAi Designer at https://rnaidesigner.thermofisher.com/maiexpress.

Alternatively or additionally, the expression of the inhibitory RNA can be controlled by a conditional promoter system which can be regulated in a dose-dependent manner by the presence of a synthetic compound, e.g. using the tetracycline-inducible promoter or a tetracycline-repressible promoter for controlling the transcription of inhibitory RNA, which is induced, resp. repressed, by administration of tetracycline.

Alternatively or additionally, the expression of MHC class I or MHC class II can be downregulated by modifying the original promoter, e.g. by causing an activity lower than the activity of the wild-type promoter controlling expression of the allogeneic or xenogeneic MHC I or of the allogeneic or xenogeneic MHC II, respectively, or the splice sites of the individual MHC genes using genome editing approaches, e.g. CRSPR/Cas technology. In HLA-A*02:01 it has been shown that the nucleotide exchange from T to C at the genomic position -101 (g-101T>C) causes a decrease in the promoter activity leading to a low expression of this allele. In HLA-B*39:01 it has been shown that the nucleotide deletion of TC at the genomic positions -151-152 (g-151-152delTC) causes a decrease in promoter activity and low expression of this allele. In HLA-A*24:92 it has been shown that the nucleotide exchange from G to A at genomic position 708 (g708G>A) causes a low expression of this allele due to an insufficient splicing. Likewise, genome editing of other promoter positions and splice sites at the intron-exon borders are capable of substantially lowering the MHC expression without abrogating expression.

For expression of complement inhibitory molecules, e.g. at least one of CD46 (SEQ ID NO: 22, SEQ ID NO: 23), CD55 (SEQ ID NO: 17, SEQ ID NO: 18, SEQ ID NO: 24) and CD59 (SEQ ID NO: 19, SEQ ID NO: 20, SEQ ID NO: 25), sequences encoding these molecules can be contained in expression cassettes, preferably under the control of a constitutive promoter. The coding sequences for complement inhibitory molecules may comprise signal peptides resulting in expression on the cell surface of the tissue.

Exemplary promoters for controlling expression of the complement inhibitory molecules are e.g. the H1 promoter (SEQ ID NO: 14), the U6 promoter (SEQ ID NO: 15), the elongation factor short (EFS) promoter (SEQ ID NO: 11), and the EF1 alpha promoter (SEQ ID NO: 12). The nucleic acid constructs for expressing inhibitory RNA directed against the allogeneic or xenogeneic MHC I and for inhibitory RNA directly against the allogeneic or xenogeneic MHC II of the tissue, and expression cassettes for expression of at least one of the complement inhibitory molecules can be contained in different nucleic acid molecules, and preferably are contained on one common nucleic acid molecule, e.g. on a DNA or RNA contained in a viral vector, which preferably is packaged in a viral particle. Preferably, the viral vector is a lentiviral vector, packaged in lentiviral particles.

The tissue can be cells, e.g. single cells, e.g. islet cells, a portion of an organ or a complete organ, which organ is e.g. a kidney, liver, heart, lung, pancreas, skin, or an extremity.

In an embodiment, the tissue of the invention can be generated by genetically manipulating a non-human mammalian animal to contain expression cassettes for downregulating expression of MHC I and MHC II, and preferably for expressing at least two of the complement inhibitory molecules. Such an animal preferably is a swine. Also in this embodiment, the expression of inhibitory RNAs for downregulating transcripts of MHC I and MHC II can be controlled by a promoter which is inducible or repressible by a synthetic compound, e.g. a tetracycline-inducible promoter, especially the Tet-on or Tet-off promoter. Accordingly, such animals can be genetically manipulated, e.g. in their germline or by somatic genetic manipulation, e.g. by viral transduction or by use of CRISPR/Cas-based methods, to contain expression cassettes encoding inhibitory RNA for downregulating MHC I and MHC II, and optionally to contain expression cassettes for expression of at least two complement inhibitory molecules, which expression cassettes contain promoters that are inducible or are repressible by a synthetic compound. Such animals preferably are transgenic for these expression cassettes. For such animals that in their germline contain the expression cassettes, integrated into the genome, it is preferred that these expression cassettes contain a promoter which is repressed by absence of the synthetic compound, e.g. a Tet-off promoter, in order to allow a MHC-expression dependant healthy development of the animals in the absence of the synthetic compound with no activity of these expression cassettes, so that after transplantation of this tissue into a recipient, activity of the expression cassettes for transcription of inhibitory RNA, and accordingly for downregulation expression of MHC I and of MHC II in the tissue can be induced by administration of the synthetic compound, e.g. tetracycline or doxycycline for use in the treatment of HvG in a recipient of the tissue. The Tet-operator element can be SEQ ID NO: 13. Alternatively, a promoter which is repressed by the presence of the synthetic compound, e.g. a Tet-on promoter, can be used, allowing a MHC-expression dependant healthy development of the animals in the presence of the synthetic compound with no activity of these expression cassettes, so that after transplantation of this tissue into a recipient, activity of the expression cassettes for transcription of inhibitory RNA, and accordingly for downregulation expression of MHC I and of MHC II in the tissue can be induced by the absence of the synthetic compound, e.g. tetracycline for use in the treatment of HvG in a recipient of the tissue. In either case, the level of expression can be adjusted to the level needed to prevent detrimental immune reactions and enable beneficial Treg cell immune reactions as described. Generally, such non-human animals are genetically manipulated to contain, by somatic genetic manipulation or preferably in their germline, nucleic acid constructs which encode inhibitory RNA under the control of a conditional promoter system which can be regulated in a dose-dependent manner by the presence of a synthetic compound, wherein inhibitory RNA under the control of the conditional promoter is arranged to downregulate expression of the MHC I by at least 50% to up to 90%, wherein inhibitory RNA under the control of the conditional promoter is arranged to downregulate expression of MHC II by at least 50% to up to 90%, wherein the downregulation of the MHC I and/or of the MHC II is in relation to its expression in wild-type tissue, and wherein the nucleic acid construct, optionally under the control of the conditional promoter, additionally expresses at least one complement inhibitory molecule. Such non-human animals can e.g. be genetically manipulated to contain nucleic acid constructs of the invention.

The invention is now described by way of examples with reference to the figures, which show in
- Fig. 1 expression levels of CD55 and of CD59 in cells that were lentivirally transduced,
- Fig. 2 PCR analytical results for integration of expression cassettes in tissue cells,
- Fig. 3 transcript levels of β2m and of MHC II in tissue cells expressing inhibitory RNA,
- Fig. 4 FACS results detecting Treg cells expanded in presence of cells with downregulated expression of MHC I and MHC II in comparison to MHC II knockout cells,
- Fig. 5 the percentage of lysed cells of tissue according to the invention which contain expression cassettes for different complement inhibitory molecules.

### Example 1: Genetic manipulation of xenogeneic pig islet cells for use as transplant

As an example for a tissue expressing xenogeneic MHC I and MHC II molecules, pig islet cells were used. For genetic manipulation of these xenogeneic cells a lentiviral vector backbone was used, which contained an expression cassette encoding according to the invention an siRNA (SEQ ID NO: 6) specific for the transcript of the Sus scrofa β2-microglobulin gene (SEQ ID NO: 30) and/or an expression cassette encoding an siRNA (SEQ ID NO: 8) specific for the transcript of the class II transactivator gene (CIITA) (SEQ ID NO: 31), preferably both expression cassettes. The inhibitory RNAs were designed and sufficient for a downregulation of MHC class I and MHC class II by 80% but not more than 90%. Following transplantation into mice as transplant recipients it was found that no cellular immunogenic response was elicited in the recipient, and no T-cell-mediated graft rejection occurred.

It was found that antibodies directed against the xenogeneic MHC I and MHC II molecules can occur, but the formation of anti-MHC I xeno-antibodies and of anti-MHC II xeno-antibodies would not result in antibody-mediated graft rejection when the suppression, i.e. downregulation, of the xenogeneic MHC I and of the xenogeneic MHC II in the tissue was by at least 80% compared to expression of the xenogeneic MHC I and MHC II, respectively, determined for wild-type pig islet cells, e.g. determined for cells treated in parallel but without genetic manipulation. For improved survival of the tissue, it was transduced additionally by a lentiviral vector which contained expression cassettes encoding complement inhibitors CD55 (SEQ ID NO: 17) and CD59 (SEQ ID NO: 19). It was found that a less intense downregulation of expression of the xenogeneic MHC I and MHC II does not result in antibody-mediated graft rejection when the xenogeneic cells expressed higher levels of complement inhibitors.

### Example 2: Genetic manipulation of allogeneic kidney for use as transplant

As an example for a tissue expressing allogeneic MHC I and MHC II molecules, a rat kidney that was genetically manipulated according to the invention was used as an allogeneic transplant in a recipient rat.

For genetic manipulation of the exemplary tissue, a nucleic acid construct was made which contained expression cassettes for a shRNA encoding SEQ ID NO: 9, which according to the invention is specifically targeting the rat β2-microglobulin mRNA (SEQ ID NO: 26), and an shRNA encoding SEQ ID NO: 10, according to the invention specifically targeting the rat MHC class II transactivator (CIITA) (SEQ ID NO: 27), as well as the coding sequences for the complement inhibitors CD55 (SEQ ID NO: 18) and CD59 (SEQ ID NO: 20). The shRNA targeting the β2-microglobulin mRNA causes silencing of MHC class I in a specific and selective manner without exceeding a level of suppression above 80% compared to untreated, i.e. wild-type, cells and the shRNA targeting the MHC class II transactivator (CIITA) causes silencing of MHC class II in a specific and selective manner without exceeding a level of suppression of 70% compared to untreated cells. The nucleic acid construct further contained an expression cassette for Luciferase, suitable for labelling cells in which the nucleic acid construct is integrated.

This nucleic acid construct in addition to shRNA targeting MHC transcripts optionally contained the coding sequences for CD55 and CD59. The nucleic acid constructs with or without the additional coding sequences for CD55 and CD59 were packaged in lentiviral particles using packaging cells, were concentrated by centrifugation at 30 000 x g for 8h and resuspended in the preservation solution. The viral particle titer was determined using p24 ELISA and the lentiviral vector particles were stored was at -80°C.

As an example for an allogeneic isolated vascularized tissue, the explanted rat kidneys of LEW1U.RT1 rats were flushed with preservation solution (composed of Custodiol) containing 80 IE heparin at a temperature of 4°C immediately after explantation. The flushed rat kidneys were connected to an ex vivo perfusion system, warmed up to 37°C and perfused for 2-3h with the vector encoding the shRNA sequences directed against MHC I and MHC II, or with the vector encoding both the shRNA sequences directed against MHC I and MHC II and expression cassettes encoding CD55 and CD59 sequences, using Custodiol supplemented with 8µg/mL Protamin sulphate and 4mg Urbason. 10⁹-10¹⁰ lentiviral particles were used per kidney. During perfusion the temperature was maintained at 37°C and a flow rate of 10-14 mL/min was used. After perfusion with the vector, the perfusion solution was replaced with Custodiol containing 3-6mL of heparinized peripheral rat blood and the kidneys were perfused for 20-30 min to remove the free residual vector. Afterwards, the kidneys were washed with fresh Custodiol solution and cooled down to 4°C.

For analyses, kidney tissue was taken from different regions and digested with collagenase VI and dispase (both obtained from Sigma). At 96h after the addition of the lentiviral vector to the second perfusion solution, endothelial cells of the kidney macrovasculature and microvasculature after labelling with anti-CD31 antibody were isolated using paramagnetic beads (Dyna-beads, obtained from Thermo Fisher) for magnetic separation. In parallel, endothelial cells were isolated from a non-transduced kidney which was treated in the same way, except that no viral particle was added. Analyses showed that endothelial cells were isolated at purities above 70%. Endothelial cells were cultivated in gelatine-coated tissue culture plates. Five days after perfusion with the lentiviral vector the MHC silencing effect and the levels of expression of CD55 and CD59 were evaluated by flow cytometric analyses.

At 24h after addition of the viral particles to the second perfusion solution, luciferase activity was detected in the culture supernatant. The levels of this bioluminescence increased during the 96h of cultivation. Remarkably, cells from the kidneys showed to be transduced, indicating integration and activity of the nucleic acid construct in the entire organ endothelium. Luciferase activity was measured after addition of luciferase substrate (Furimazine, Promega).

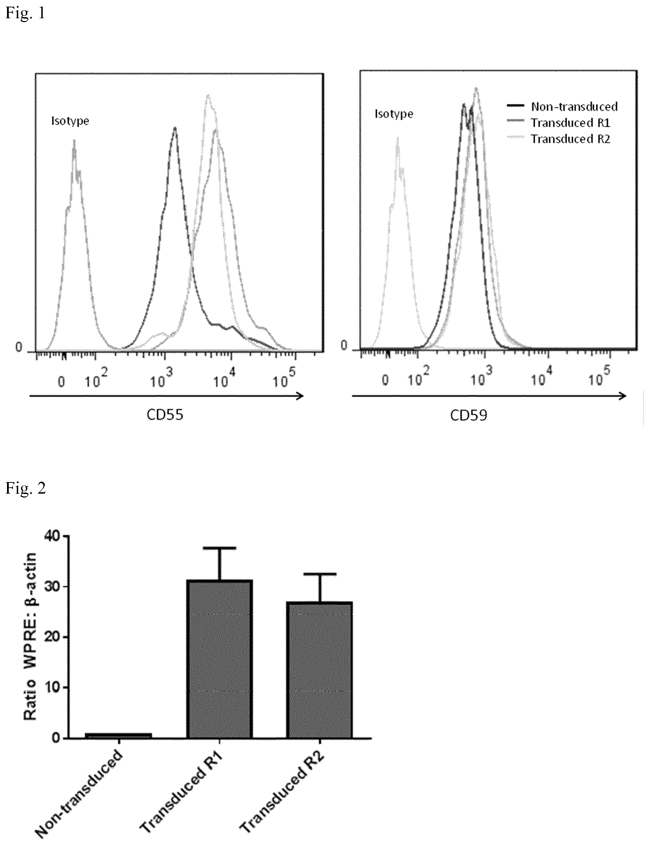

Fig. 1 depicts the expression levels of CD55 and CD59 in rat kidney endothelial cells isolated from two different regions designated R1 and R2 of the kidney perfused with the lentiviral vector encoding for CD55 and CD59 in comparison to the native levels expressed in a kidney perfused without vector (non-transduced). The result shows that lentiviral particles provided for stable and permanent integration the nucleic acid construct including the expression cassettes into the genome of the vascularized tissue.

Fig. 2 depicts the results of real-time PCR using DNA that was isolated from the cultivated rat endothelial cells and primers that were specific for the woodchuck hepatitis virus posttranscriptional element WPRE, which is present in the nucleic acid construct. The result shows the high levels of WPRE, respectively of the nucleic acid construct according to the invention, in the endothelial cells treated by the method of the invention, and background levels only in the endothelial cells from the kidneys that were treated in parallel but without addition of the lentiviral vector

Isolated cells were cultured in EBM-2 endothelial cell growth medium (obtained from Lonza). Endothelial cells were stimulated with IFN-γ (100ng/mL) for 48h. It was found that endothelial cells from the microvasculature of the non-transduced parallel treatment were able to up-regulate MHC class I (RT1-A) and MHC class II (RT1-B) transcript levels (p<0.001) upon exposure to IFN-γ, but microvascular endothelial cells from kidneys treated with the nucleic acid construct according to the invention, contained in the lentiviral particle, upon IFN-γ stimulation did not show a significant increase in transcript levels for β2-microglobulin and RT1-B. These results demonstrate that the method of the invention effectively silences expression of MHC class I and of MHC class II in the vascularized tissue and leads to an expression of the transgenes CD55 and CD59. Also, no ischemic damage was found, which could be caused by undersupply with oxygen.

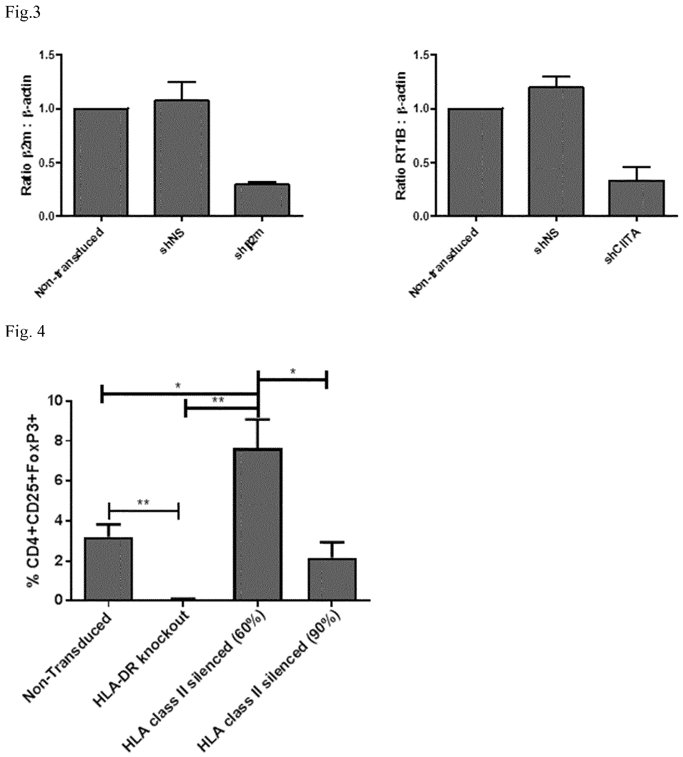

Fig. 3 depicts the levels detected by RT-PCR for transcripts of β2-microglobulin and for transcripts of MHC class II (RT1-B) after stimulation with IFNγ for 48h in the cultivated endothelial cells. As a negative control, non-transduced cells were used and cells that were transduced with a vector encoding a non-binding non-specific shRNA (shNS). Transcript levels are normalized to levels of transcripts for β-actin (***p<0.001), also detected by RT-PCR. These results show that the nucleic acid construct encoding shRNA specific for transcripts of β2-microglobulin, respectively for transcripts of CIITA, effectively downregulate expression of β2-microglobulin and of CIITA, and hence downregulate expression of MHC I and MHC II.

Fig. 4 depicts the analytical result of FACS for Treg cells (CD4+CD25+FoxP3+). The result shows that frequencies of Treg cells that were expanded in the presence of cells containing an expression cassette for shRNA directed against MHC II transcripts for downregulation by 60% (HLA class II silenced (60%)) or by 90% (HLA class II silenced (90%)), are higher than the frequencies of Tregs in the presence of MHC class II knockout cells (HLA-DR knockout, 100% downregulated) and in the moderate downregulation of MHC class II transcripts by 60% even higher than with non-transduced cells. HLA-DR knockout single donor HUVEC cells or HLA class II silenced single donor HUVEC cells that were downregulated for up to 60% or up to 90% were co-cultured in presence of PBMCs in RPMI medium supplemented with 5% AB serum and 100U/mL IL-2 and 20ng TGF-β. Non-transduced cells were used as controls (Non-Transduced).

For allogeneic transplantation, the kidneys from explanted kidneys of LEW1U.RT1 donor rats were used as allogeneic transplants in LEW.RT1 rats as recipients. The right kidney was perfused and genetically manipulated as described above and then was transplanted in rat LEW.RT1 recipients in an orthotopic manner. During the operation the animals are maintained on a thermoplate to maintain the body temperature constant. The recipient was ventilated via a respiratory mask. For anaesthesia, Isofluran was administered. After laparotomy the right kidney was resected and the vessels clamped. The genetically modified or unmodified control donor kidneys, respectively, was meanwhile cooled in the ex vivo perfusion system. The kidney was removed from the ex vivo perfusion system, flushed with fresh cold perfusion solution and prepared for implantation. The kidney was then transplanted using standard techniques. After discontinuation of anesthesia and removal of the respiratory mask, the animals were put into boxes provided with drinking water. The animals were regularly controlled for signs of rejection and cytokine levels such as IP-10, MCP-1 or RANTES were monitored. In the first 14 days after transplantation the animals received a mild immunosuppression (10mg/kg body weight Cyclosporin/day). Animals that were transplanted with a genetically modified organ did not show clinical, immunological or morphological signs of immunological organ rejection within an observation period of 90 days, whereas animals that were transplanted with an unmodified kidney rejected the graft at the latest 30 days after transplantation.

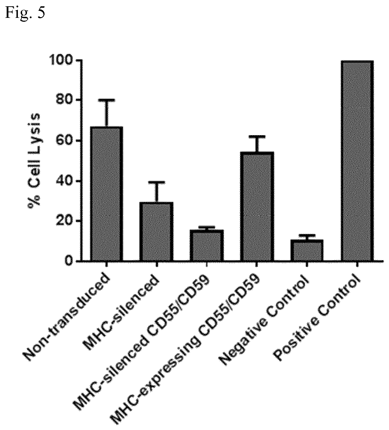

Fig. 5 depicts the percentage of lysed cells which contain expression cassettes for different complement inhibitory molecules. The results show that the overexpression of CD55 and CD59 in MHC-silenced rat endothelial cells (LEW.1W) substantially decreases antibody-mediated cytotoxicity nearly to the level of the negative control. Rat endothelial cells (ECs) were incubated with serum of an immunized rat containing anti-RT1^{u} antibodies and complement. MHC-silenced ECs overexpressing CD55 and CD59 were best protected against complement-dependent antibody-mediated cell lysis. Only MHC-silenced ECs showed an improved protection compared to non-MHC-silenced only CD55 and CD59 transgenes expressing ECs. The data was normalized to the positive control. The negative control used serum from the EC donor (autologous control).

This example shows that the transplantation of an allogeneic kidney does not induce an immunogenic cellular reaction by the recipient and does not induce a T-cell and B-cell-mediated rejection (HvG), which kidney was genetically manipulated by retroviral transduction to contain expression cassettes for downregulating expression of its MHC I and MHC II by at least 80%, leaving a residual expression of at most 20%, compared to wild-type expression, and to contain expression cassettes for expression of complement inhibitors CD55 an CD59.

## Claims

1. Tissue for use as a transplant, which tissue expresses allogeneic or xenogeneic MHC I and/or allogeneic or xenogeneic MHC II molecules,
wherein the expression of the allogeneic or xenogeneic MHC I is downregulated by at least 50% to up to 90%,
wherein the expression of the allogeneic or xenogeneic MHC II is downregulated by at least 50% to up to 90%, wherein the downregulation of the allogeneic or xenogeneic MHC I and/or of the allogeneic or xenogeneic MHC II is in relation to its expression in wild-type tissue,
and wherein the tissue additionally expresses at least one complement inhibitory molecule on the tissue.

2. Tissue for use as a transplant according to claim 1, **characterized in that** the expression of the allogeneic or xenogeneic MHC I is downregulated by 80% up to 90%, and wherein the expression of the allogeneic or xenogeneic MHC II is downregulated by at least 80% to up to 90%.

3. Tissue according to one of the preceding claims, **characterized in that** the expression of the allogeneic or xenogeneic MHC I and/or the expression of the allogeneic or xenogeneic MHC II is downregulated by the tissue being genetically manipulated to contain at least one nucleic acid construct comprising at least one expression cassette encoding inhibitory RNA interfering with at least one transcript encoding the allogeneic or xenogeneic MHC I molecule and/or encoding the allogeneic or xenogeneic MHC II molecule.

4. Tissue according to one of the preceding claims, **characterized in that** the expression of the allogeneic or xenogeneic MHC I is downregulated by the tissue being genetically manipulated to contain a nucleic acid construct comprising at least one expression cassette encoding an siRNA which hybridizes to the transcript of the β2-microglobuline gene (β2m), and/or encoding an siRNA which hybridizes to the transcript of at least one, or two, preferably for transcripts of all three heavy chains of HLA-A, HLA-B and HLA-C molecules or SLA-1, SLA-2 and SLA-3 molecules.

5. Tissue according to one of the preceding claims, **characterized in that** the expression of the allogeneic or xenogeneic MHC II is downregulated by the tissue being genetically manipulated to contain a nucleic acid construct comprising at least one expression cassette encoding an siRNA which hybridizes to transcripts of at least one of the alpha chain and/or beta chain of HLA-DR, HLA-DQ and/or of HLA-DP or SLA-DR and/or SLA-DQ, and/or by encoding an siRNA which is specific for transcripts of the class II transactivator gene.

6. Tissue according to one of the preceding claims, **characterized in that** the complement inhibitory molecule is selected from CD46, CD55 and CD59.

7. Tissue according to one of the preceding claims, **characterized by** excluding or reducing immunosuppressants for permanent use in the treatment of HvG disease.

8. Tissue according to one of the preceding claims, **characterized in that** the tissue is a portion of an organ or of an extremity or a complete organ or extremity.

9. Non-human mammalian animal which is genetically manipulated to comprise tissue according to one of the preceding claims.

10. Non-human animal according to claim 9, **characterized in that** the animal is genetically manipulated to contain, in its germline or by somatic genetic manipulation, nucleic acid constructs which encode inhibitory RNA under the control of a conditional promoter system which can be regulated in a dose-dependent manner by the presence of a synthetic compound, wherein inhibitory RNA under the control of the conditional promoter is arranged to downregulate expression of the MHC I by at least 50% to up to 90%,
wherein inhibitory RNA under the control of the conditional promoter is arranged to downregulate expression of MHC II by at least 50% to up to 90%, wherein the downregulation of the MHC I and/or of the MHC II is in relation to its expression in wild-type tissue,
and wherein the nucleic acid construct, optionally under the control of the conditional promoter, additionally expresses at least one complement inhibitory molecule.

11. Process for producing a tissue expressing an MHC I and an MHC II for use as a transplant, comprising providing an isolated tissue with a nucleic acid construct that comprises at least one expression cassette encoding at least one siRNA set up to downregulate the expression of the MHC I by at least 50% to up to 90% in relation to its expression in wild-type tissue, and comprises at least one expression cassette encoding at least one siRNA set up to downregulate the expression of the MHC II by at least 50% to up to 90% in relation to its expression in wild-type tissue, and comprising providing the isolated tissue with a nucleic acid construct containing an expression cassette encoding at least one complement inhibitory molecule.

12. Process according to claim 11, **characterized in that** the MHC I and MHC II are allogeneic or xenogeneic for a recipient of the transplant.

13. Nucleic acid construct for use in the treatment of HvG disease or for use in a process of one of claims 9 to 12, **characterized in that** the nucleic acid construct comprises at least one expression cassette encoding an siRNA set up to downregulate expression of an MHC I of a tissue by at least 50% to up to 90%, at least one expression cassette encoding an siRNA set up to downregulate expression of an MHC II of a tissue by at least 50% to up to 90%, and expression cassettes encoding at least one complement inhibitory molecule.

14. Nucleic acid construct according to claim 13, **characterized in that** it is comprised of at least two nucleic acid molecules.

15. Nucleic acid construct according to claim 13, **characterized in that** it is comprised of one nucleic acid molecule.
